# EUROPEAN PATENT APPLICATION

(11) **EP 0 852 142 A1**
(43) Date of publication of application: **08.07.1998**
(21) Application number: 98200466.5
(22) Date of filing: 14.05.1993
(51) Int. Cl.: A61K 31/135, C07C 211/53, C07C 233/36, C07C 233/78, C07C 275/32, C07D 213/46, A61K 31/165, A61K 31/21

(54) **Diamines in the treatment of arrhythmia**

(30) Priority: 14.05.1992 FR 9205855
(62) Divisional of application: 93909965.1
(71) Applicant: SMITHKLINE BEECHAM LABORATOIRES PHARMACEUTIQUES, 92731 Nanterre Cédex (FR)
(72) Inventor: Bril, Antoine M A., SmithKline Beecham Lab. Pharm., 35762 Saint Grégoire (FR); Faivre, Jean-François S P., SmithKline Beecham, 35762 Saint Grégoire (FR); Forest, Marie-Claire, SmithKline Beecham, 35762 Saint Grégoire (FR); Gout, Bernard Emile J., SmithKline Beecham, 35762 Saint Grégoire (FR)
(74) Representative: Rutter, Keith, Dr.

(57) **Abstract**

**NOVEL TREATMENT**

A novel treatment of arrhythmia in human or non-human mammals, comprising administering a compound having combined class III and class IV antiarrhythmic activity.

## Description

This invention relates to a novel treatment and in particular to a novel treatment for arrhythmia and to certain novel compounds and compositions used in such treatment.

Antiarrhythmic drugs have been grouped according to the pattern of electrophysiological effects they produce and/or their presumed mechanism of action. A classification in four groups has been originally proposed by Vaughan Williams in 1970. At the molecular level, class I compounds act on Na⁺ currents; class II compounds possess b-adrenoceptor blocking activity; class III drugs block K⁺ channels; and class IV drugs target Ca⁺⁺ channels.

European Patent Application, Publication Number 0233762 discloses compounds of formula (I) therein which are stated to have cardiovascular activity, including use in the treatment of angina.

It has now surprisingly been discovered that certain compounds of formula (I) of EP0233762 show potential as antiarrhythmic agents: This antiarrhythmic activity is considered to be associated with an increase in the QT interval.

Certain of the compounds of formula (I) surprisingly evidenced a prolongation of the cardiac action potential and are therefore considered to show particular promise as class III antiarrhythmic agents.

Most surprisingly however, certain of the compounds of formula (I) of EP0233762 demonstrated both class III and class IV activity, the class IV activity providing the compounds with a self limiting increase of the cardiac cell action potential duration. These compounds therefore show a improved profile over pure class III agents, having less proarrhythmic potential, and also a lack of cardiodepressive activity in ischaemic myocardium. These compounds are also considered to be effective against atrial fibrillation and flutter.

One compound in particular demonstrates such dual activity, this is the compound of Example 50 of EP-A-8603765 named as (N-[3,4-dimethoxy-phenyl]-N-3-[N'-2-(3,4-dimethoxyphenyl)-ethyl-N'-methylamino]propyl)-N'-4-nitrobenzamide, (also refereed to herein as 'Compound I').

Accordingly, the present invention provides a method for the treatment of arrhythmia in human or non-human mammals, which method comprises the administration of a effective, non-toxic amount of a compound of formula (I): 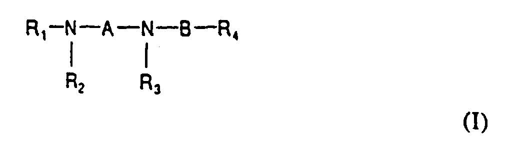 or a pharmaceutically acceptable salt thereof and/or a pharmaceutically acceptable solvate thereof,
wherein
R₁ and R₄ are independently phenyl optionally substituted by one, two or three of halogen, C₁₋₄ alkoxy, C₁₋₄ alkyl, cyano, hydroxy, nitro, NR₅R₆ or O₂SNR₅R₆ wherein R₅ and R₆ are independently hydrogen or C₁₋₆ alkyl or together are C₃₋₆ polymethylene, or disubstituted at adjacent carbon atoms by C₁₋₂ alkylenedioxy and optionally further substituted by one of the above groups;
R₂ is selected from (CH₂)_{z} CN where z is O or an integer from 1 to 4, C₁₋₁₂ alkyl, C₃₋₇ cycloalkyl, C₃₋₇ cycloalkyl C₁₋₄ alkyl, phenyl C₁₋₄ alkyl, pyridyl, pyridyl C₁₋₄ alkyl, COR₇, COCH₂COR₇, SO₂R₇, CO₂R₇, CONHR₇ and CSNHR₇, where
R₇ is selected from C₁₋₁₂ alkyl, C₃₋₇ cycloalkyl, C₃₋₇ cycloalkyl C₁₋₄ alkyl, phenyl and phenyl C₁₋₄ alkyl, any alkyl moiety in R₇ optionally substituted by hydroxy or C₁₋₄ alkanoyloxy, any pyridyl or phenyl moiety in R₂ optionally substituted as defined for R₁ and R₄ and any cycloalkyl moiety in R₂ optionally substituted by one or two C₁₋₄ alkyl groups;
R₃ is hydrogen, C₂₋₆ alkyl, C₂₋₆ alkene or C₃₋₈ cycloalkyl;
A represents C₂₋₅ alkylene; and
B represents C₁₋₄ alkylene.

In a further aspect, the present invention provides the use of a compound of formula (I) or a pharmaceutically acceptable salt thereof and/or a pharmaceutically acceptable solvate thereof, for the manufacture of a medicament for the treatment of arrhythmia.

The invention further provides a pharmaceutical composition for the treatment of arrhythmia, which comprises a compound of formula (I) or a pharmaceutically acceptable salt thereof and/or a pharmaceutically acceptable solvate thereof and a pharmaceutically acceptable carrier therefore.

Arryhthmia particularly includes cardiac arrhythmia and ischaemic rhythm disorders, especially cardiac arrhythmia.

Suitable values for R₁ and R₄ include phenyl, phenyl di-substituted by methylenedioxy and optionally further substituted by chloro, or phenyl substituted by one of fluoro, chloro or bromo or by one, two or three of methoxy, ethoxy, n- or iso-propoxy, methyl, ethyl, n - or iso-propyl, cyano, hydroxy or amino optionally substituted by one or two methyl groups.

R₁ and R₄ are preferably the same group, most preferably 3,4-dimethoxyphenyl.

Particular values for R¹ and R⁴ are halophenyl, especially 4-halophenyl, dihalophenyl, especially 3,4-dihalophenyl, dialkylphenyl, especially 3,4-dialkoxyphenyl, (hydroxy, alkoxy)phenyl such as (3-alkoxy,4-hydroxy)phenyl and trialkoxyphenyl such as 3,4,5-trialkoxyphenyl.

Examples of R¹ and R⁴ include 4-fluorophenyl, 3,4-dichlorophenyl, 3,4-dimethoxyphenyl, (3-methoxy, 4-hydroxy)phenyl and 3,4,5-trimethoxyphenyl.

Phenyl moieties in R₂ include phenyl, 2-,3- and 4-nitrophenyl, 3,5-dinitrophenyl, 3-methoxyphenyl, 3-methoxy-6-methylphenyl, 2,4,6-trimethylphenyl, 4-cyanophenyl, 4-chlorophenyl and 4-methylphenyl.

Suitable values for alkylene moieties in R₂ include -CH₂-, -CH(CH₃)--CH₂CH₂CH₂- and -CH₂CH₂-. Suitable values for R₂ when alkyl include n-pentyl.

Suitable values for alkyl R₇ in R₂ includes C₁₋₆ alkyl, such as methyl, ethyl, n- and is- C₃H₇, n-C₄H₉, n-C₅H₁₁, nC₆H₁₃, nC₇H₁₅, nC₈H₁₇ and nC₁₁H₂₃.

Suitable values for pyridyl moieties in R₂ include 2-and 4 - pyridyl.

Suitable values for optional substituents on alkyl moieties in R₇ include acetoxy.

Suitable values for z in R₂ include 0 or 1.

Suitable values for C₁₋₄ alkyl groups in R₂ include methyl and ethyl.

Suitable values for R₃ include hydrogen, methyl, ethyl, n- and iso-propyl, and n, iso -, sec - and t-butyl.

Preferably R₃ is methyl.

Suitable values for A and B include -(CH₂)₂- and -(CH₂)₃-.

Preferably, A is -(CH₂)₃-.

Preferably, B is -(CH₂)₂-.

There is a group of compounds within formula (I) of formula (IA): 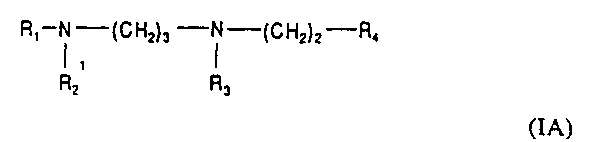 wherein R₂¹ is CONHR₇ Or CSNHR₇ where R₇ is as defined in formula (I), and the remaining variables are as defined in formula (I).

There is another group of compounds within formula (I) of formula (IB): 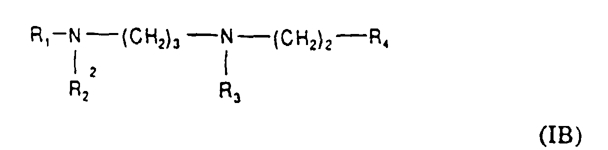 wherein R₂² is COR₇ where R₇ is as defined in formula (I), and the remaining variables are as defined in formula (I).

Suitable and preferred values for the variables of formulae (IA), and (IB) are as described for the corresponding variables under formula (II).

There is a preferred group of compounds within formula (IB) which are characterised in that R₇ is C₁₋₁₂ alkyl.

The compounds of formula (IB) wherein R₇ is C₁₋₁₂ alkyl, or pharmaceutically acceptable salts thereof and/or a pharmaceutically acceptable solvate thereof, are class III antiarrhythmic agents, and hence can be used as such, for example they are effective against ventricular fibrillation.

There is a particularly preferred group of compounds within formula (IB) which are characterised in that R₇ is optionally substituted phenyl, optional substituents being selected from one halogen atom, especially a chlorine atom, and one, two or three of cyano, hydroxy, nitro and NR₅R₆ wherein R₅ and R₆ are independently hydrogen (especially)or C₁₋₆ alkyl; a preferred substituent is a nitro group, especially a 4-nitro group.

The compounds of formula (IB) wherein R₇ is optionally substituted phenyl, optional substituents being selected from one halogen atom, especially a chlorine atom, and one, two or three of cyano, hydroxy, nitro and NR₅R₆ wherein R₅ and R₆ are independently hydrogen (especially)or C₁₋₆ alkyl, or pharmaceutically acceptable salts thereof and/or a pharmaceutically acceptable solvates thereof, are combined class III and class IV antiarrhythmic agents and hence can be used as such, for example they are particularly effective against ventricular fibrillation, they are also considered to be effective against atrial fibrillation and flutter.

In its most preferred aspect, the compound of formula (I) is that compound wherein R₁ represents 3,4-dimethoxyphenyl, R₂ represents a 4-nitrobenzoyl group, R₃ represents methyl, R₄ represents 3,4-dimethoxyphenyl, A represents (CH₂)₃ and B represents (CH₂)₂: i.e.the compound of example 50 of EP 0233762.

Where the compounds of formula (I) possess chiral carbon atoms (for example when A and/or B are branched alkylene) they may exist in more than one stereoisomeric form, the invention extends to all such stereoisometric forms, including enantiomers of the compounds of formula (I) and to mixtures thereof, including racemates.

The different stereoisomeric forms may be separated or resolved one from the other by the usual methods or any given isomer may be obtained by stereospecific or asymmetric syntheses.

When used herein alkyl, alkene and alkylene includes straight- and branched-chain alkyl, alkene and alkylene groups, each with up to 12, suitably up to 6 carbon atoms, unless stated to the contrary.

Pharmaceutically acceptable salts include acid addition salts with conventional acids such as hydrochloric, hydrobromic, boric, phosphoric, sulphuric and pharmaceutically acceptable organic acids such as acetic, tartaric, maleic, citric, succinic, benzoic, ascorbic, methanesulphonic, a-keto-glutaric, a-glycerophosphoric, and glucose-1-phosphoric acids. Preferably the acid addition salt is a hydrochloride; this is conveniently prepared by treating the relevant compound with HCl in a solvent such as diethylether.

Pharmaceutically acceptable salts also include quaternary salts. Examples of quaternary salts include such compounds quaternised by compounds such as R₈-T wherein R₈ is C₁₋₆ alkyl, phenyl-C₁₋₆ alkyl or C₅₋₇ cycloalkyl, and T is a radical corresponding to a anion of an acid. Suitable examples of R₈ include methyl, ethyl and n- and iso- propyl; and benzyl and phenethyl. Suitable T include halide such as chloride, bromide and iodide.

Pharmaceutically acceptable salts also include pharmaceutically acceptable N-oxides, and the invention extends to these.

The compounds of the formula (I) and their pharmaceutically acceptable salts may also form solvates with pharmaceutically acceptable solvates and the invention extends to these.

It will also be realized that salts of the compounds of the formula (I) which are not pharmaceutically acceptable may be useful as intermediates in the preparation of pharmaceutically acceptable salts of compounds of the formula (I) or the compounds of the formula (I) themselves, and as such form an aspect of the present invention.

Certain of the compounds of formula are considered to be novel. Such compounds therefore form a further part of the present invention.

Accordingly, there is provided a compounds of formula (IC): 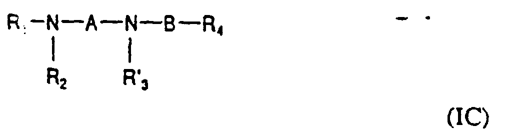 or a pharmaceutically acceptable salt thereof and/or a pharmaceutically acceptable solvate thereof, wherein
R₁, R₂, A, B and R₄ are as defined in relation to formula (I), and R'₃ represents C₂₋₆ alkene or C₃₋₈ cycloalkyl.

The invention also provides a pharmaceutical composition comprising a compound of formula (IC) or a pharmaceutically acceptable salt thereof and/or a pharmaceutically acceptable solvate thereof, and a pharmaceutically acceptable carrier.

The invention also provides a compound of formula (IC) or a pharmaceutically acceptable salt thereof and/or a pharmaceutically acceptable solvate thereof, for use as an active therapeutic substance, in particular for use in the treatment of arrhythmia.

There are also certain compounds of formula (I) and the pharmaceutically acceptable salts thereof and/or the pharmaceutically acceptable solvates thereof which have not been disclosed in EP-A-0233762 and which are considered to be novel and form part of the present invention.

Accordingly, there is provided:
N-[2-(3,4-dimethoxyphenyl)ethyl]-N'-[3-[[2-(3,4-dimethoxyphenyl)ethyl]methyl-amino]propyl] -4-nitrobenzamide,
N-(4-fluorophenyl)-N'-[3-[(2-(3,4-dimethoxyphenyl)ethyl] methylamino]propyl]-4-nitrobenzamide,
N-(3,4-dichlorophenyl)-N-[3-[[2-(3,4-dimethoxyphenyl)ethyl] methylamino]propyl] -4-nitrobenzamide,
N-(3,4,5-trimethoxyphenyl)-N-[3-[[2-(3,4-dimethoxyphenyl)ethyl] methylamino]propyl] -4-nitrobenzamide,
N-(3,4-dimethoxyphenyl)-N-[3-[[2-(3,4-methoxyphenyl)ethyl]-2-propenylamino]propyl]-4-nitrobenzamide,
4-amino-N-(3,4-dimethoxyphenyl)-N-[3-[[2-(3,4-dimethoxyphenyl)ethyl]methyl-amino] propyl]benzamide,
N-[3-[[2-(3,4-dimethoxyphenyl)ethyl]methylamino]propyl]-N-[3,4-bis(1-methylethoxy) phenyl]-4-nitrobenzamide,
N-(3,4-dimethoxyphenyl)-N-[3-[[2-(3,4-dimethoxyphenyl)ethyl](1-methylethyl)amino]propyl]-4-nitrobenzamide,
N-(3,4-diethoxyphenyl)-N-[3-[[2-(3,4-Diethoxyphenyl)ethyl]methylamino]propyl]-4-nitrobezenecarboxamide,
N-(3,4-dimethoxyphenyl)-N-[3[[2-(3,4-dimethoxyphenyl)ethyl]cyclopropylamino]propyl]-4-nitrobenzamide,
N-(4-hydroxy-3-methoxyphenyl)-N-[3[[2-(3,4-dimethoxyphenyl)ethy]-methylamino]propyl]-4-nitrobenzamide, and
N-(3,4-dimethoxyphenyl)-N-[3-[[2-(3,4-dimethoxyphenyl)ethyl]methylamino]propyl]-4-pyridinecarboxamide, (each of which which may be referred to hereinafter as a 'Novel Compound'); or a pharmaceutically acceptable salt thereof and/or a pharmaceutically acceptable solvate thereof.

The invention also provides a pharmaceutical composition comprising a Novel Compound or a pharmaceutically acceptable salt thereof and/or a pharmaceutically acceptable solvate thereof, and a pharmaceutically acceptable carrier.

The invention also provides a Novel Compound or a pharmaceutically acceptable salt thereof and/or a pharmaceutically acceptable solvate thereof, for use as an active therapeutic substance, in particular for use in the treatment of arrhythmia.

Pharmaceutically acceptable salts include conventional salts such as those disclosed in EP-A-0233762, but in particular the hydrochloride.

Pharmaceutically acceptable solvates include hydrates.

The compounds of formula (I), pharmaceutically acceptable salts thereof and/or a pharmaceutically acceptable solvates thereof may be prepared according to known methods including those methods disclosed in EP-A-0233762. In particular, the compound wherein R₁ represents 3,4-dimethoxyphenyl, R₂ represents a 4-nitrobenzoyl group, R₃ represents methyl, R₄ represents 3,4-dimethoxyphenyl, A represents (CH₂)₃ and B represents (CH₂)₂ may be prepared according to methods disclosed in Example 50 of EP-A-0233762.

EP-A-0233762 also discloses certain intermediates for preparing the compounds of formula (I) which intermediates have the formula (II):

R₁ - NH - A - NR₃ - B - R₄ (II)

wherein R₁, A, R₃, B and R₄ are as defined in relation to formula (I).

Surprisingly, it has been discovered that the compounds of formula (I) show potential as an antiarrhythmic agents.

Accordingly, the present invention also provides a pharmaceutical composition comprising a compound of the above defined formula (I) and a pharmaceutically acceptable carrier therefor.

The invention also provides a compound of formula (II) or a pharmaceutically acceptable salt thereof and/or a pharmaceutically acceptable solvate thereof, for use as a therapeutically active compound.

In particular, the invention provides a compound of formula (II) or a pharmaceutically acceptable salt thereof and/or a pharmaceutically acceptable solvate thereof, for use in the treatment of arrhythmia.

In a further aspect, the present invention provides a method for the treatment of arrhythmia in human or non-human mammals, which method comprises the administration of an effective, non-toxic amount of a compound of formula (II) or a pharmaceutically acceptable salt thereof and/or a pharmaceutically acceptable solvate thereof.

The invention also provides the use of a compound of formula (II) or a pharmaceutically acceptable salt thereof and/or a pharmaceutically acceptable solvate thereof, for the manufacture of a medicament for the treatment of arrhythmia.

Particular, suitable and preferred values for the variables R₁, A, R₃, B and R₄ in the compound of formula (II) are as defined herein in regard to the compounds of formula (I).

A compound of formula (II) or a pharmaceutically acceptable salt thereof and/or a pharmaceutically acceptable solvate thereof may be prepared according to known methods including those methods disclosed in EP-A-0233762.

As indicated above compounds of formula (I) and (II) and the pharmaceutically acceptable salts thereof and/or the pharmaceutically acceptable solvates thereof (the active compounds) are of use in medicine.

Active compounds or a pharmaceutically acceptable salt thereof and/or a pharmaceutically acceptable solvate thereof is normally administered in unit dosage form.

An amount effective to treat the disorder hereinbefore described depends upon such factors as the efficacy of the active compounds , the particular nature of the pharmaceutically acceptable salt or pharmaceutically acceptable solvate chosen, the nature and severity of the disorders being treated and the weight of the mammal. However, a unit dose will normally contain 0.1 to 500 mg for example 2 to 50 mg, of the compound of the invention. Unit doses will normally be administered once or more than once a day, for example 2,3,4,5 or 6 times a day, more usually 2 to 4 times a day, such that the total daily dose-normally in the range, for a 70 kg adult of 0.1 to 2500 mg, more usually 50 to 2000 mg, for example 10 to 75mg, that is in the range of approximately 0.002 to 35 mg/kg/day, more usually 1 to 30 mg/kg/day, for example 0.15 to 1 mg/kg/day.

At the above described dosage range, no toxicological effects are indicated for the compounds of the invention.

In such treatment, the active compound may be administered by any suitable route, e.g. by the oral, parenteral or topical routes. For such use, the compound will normally be employed in the form of a pharmaceutical composition in association with a human or veterinary pharmaceutical carrier, diluent and/or excipient, although the exact form of the composition will naturally depend on the mode of administration.

Compositions are prepared by admixture and are suitably adapted for oral, parenteral or topical administration and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents,

For parenteral administration, fluid unit dose forms are prepared containing a compound of the present invention and a sterile vehicle. The compound depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

For topical administration, the composition may be in the form of a transdermal ointment or patch for systemic delivery of the active compound and may be prepared in a conventional manner, for example, as described in the standard textbooks such as 'Dermatological Formulations' - B.W. Barry (Drugs and the Pharmaceutical Sciences - Dekker) or Harrys Cosmeticology (Leonard Hill Books).

In addition such compositions may contain further active agents such as anti-hypertensive agents and diuretics.

As is common practice, the compositions will usually be accompanied by written or printed directions for use in the medical treatment concerned.

No toxicological effects are indicated when an active compound is administered in the above mentioned dosage ranges.

The following, descriptions, examples and pharmacological methods illustrate the invention but do not limit it in any way.

### Description 1

### Ethyl [3-[2-[2-(3,4-dimethoxyphenyl)ethyl]methylamino]-propionate

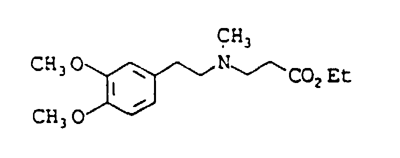

A solution of 7.8 g, (40 mmol) of 3,4-dimethoxy-N-methylbenzeneethanamine ,5.5 g, (40 mmol) of ethyl 3-chloropropionate , and 4.3 g, (42 mmol) of triethylamine in 150 ml of acetonitrile was refluxed for two and half hours. After cooling to room temperature, the reaction mixture was concentrated *in vacuo*. The resulting crude product was dissolved in ethyl acetate and washed successively with water and brine. The organic layer was dried over MgSO₄ and concentrated *in vacuo* to dryness, affording 10.7 g (90%) of a pure yellow oil.
¹H NMR (CDCl₃) δ = 1.25 (t,3H,J=7Hz,CH₃); 2.32 (s,3H,NCH₃); 2.40-2.85 (m,8H,(CH2)x4); 3.85 (s,3H,OCH₃); 3.88 (s,3H,OCH₃); 4.13 (q,2H,J=7Hz,CH₂); 6.65-6.85 (m,3H,Ar) ppm.

### Description 2

### N-[2-(3,4-Dimethoxyphenyl)ethyl]-3-[2-(3,4-dimethoxyphenyl)ethyl]methylamino]propanamide

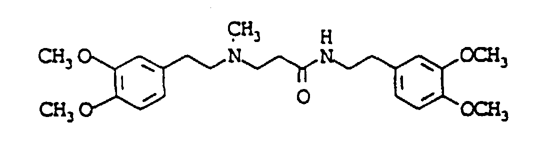

A mixture of 1.5 g (5 mmol) of ethyl [3-[2-[2-(3,4-dimethoxyphenyl)ethyl]methylamino]-propionate (D1) and 0.9 g (5 mmol) of (3,4-dimethoxy)-benzeneethanamine was warmed up to 150°C under argon for 18 hours, in the presence of a catalytic amount of trimethylaluminium. After cooling down to room temperature, the reaction mixture was diluted with ethyl acetate and washed with water and treated with a 5% aqueous HCl. The aqueous layer was separated and successively washed with ethyl acetate, basified with 5N aqueous NaOH, and extracted with ethyl acetate. The organic layer was separated, washed with brine, dried over MgSO₄ and concentrated *in vacuo*. Purification of the crude product by flash chromatography on silica gel using 97:3 methylene chloride:methanol afforded 600 mg (30%) of a yellow amorphous solid.
¹H NMR (CDCl₃) δ = 2.20 (s,3H,NCH₃); 2.25-2.95 (m,10H,CH₂x5); 3.35 (m,2H,CH₂); 3.81 (s,3H,OCH₃); 3.82 (s,3H,OCH₃); 3.87 (s,3H,OCH₃): 3.88 (s,3H,OCH₃); 6.60-6.85 (m,6H,Ar); 8.12 (m,1H,exch.D₂O,NH) ppm.

### Description 3

### 3-Chloro-N-(4-fluorophenyl)-propanamide

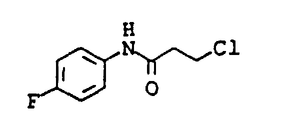

To a solution of 5.00 g (45 mmol) of 4-fluorobenzeneamine and 4.88 g (56 mmol) of triethylamine in 30 ml of methylene chloride at 0°C,was added dropwise 5.72 g (45 mmol) of 3-chloropropionyl chloride neat or dissolved in 5 ml methylene chloride and the reaction mixture was allowed to stir at room temperature for 1 hr.The reaction mixture was then washed with 100 ml water, 100 ml of 0.5N HCl, and then with water until neutral,
The organic phase was separated, dried over MgSO₄ ad concentrated to afford 5.51 g (60%) of a pale pink product which was purified by trituration with diisopropyl ether.
m.p. 106-107°C.

### Description 4

### N-(4-Fluorophenyl)-3-[[2-(3,4-dimethoxyphenyl)ethyl]methylamino]-propanamide

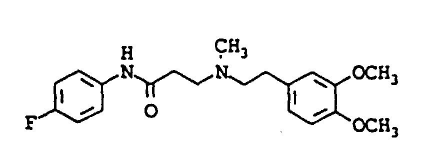

To a stirred solution of 4.22 g (20 mmol) of 3-chlor-N-(4-fluorophenyl)-propanamide (D3) and 2.1 g (26 mmol) of triethylamine in 50 ml of acetonitrile, 4.08 g (21 mmol) of 3,4-dimethoxy-N-methylbenzeneethanamine were added and the reaction mixture was refluxed for 24 hours.
After cooling the reaction mixture was concentrated to dryness.

150 ml of ethyl acetate were added and the solution was washed with 100 ml water. The organic phase was separated, dried over MgSO₄ and concentrated *in vacuo* affording 1.09g (14%) of an orange oil. This was purified by flash chromatography on silica gel using 95:5 ethyl acetate : methanol.

### Description 5

### N-(4-Fluorophenyl)-N'-[2-(3,4-dimethoxyphenyl)ethyl]-N'-methyl-1,3-propanediamine

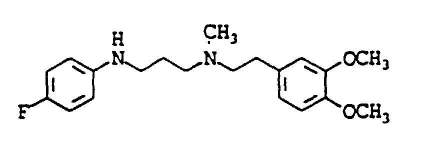

To a stirred solution of 1.0 g (2.7 mmol) of N-(4-fluorophenyl)-3-[[2-(3,4-dimethoxyphenyl) ethyl]methylamino]-propanamide (D4) in 20 ml of tetrahydrofuran, 0.15 g of LiAlH₄ (4.1 mmol) was added cautiously and the reaction mixture was refluxed for 4 hours.
A further 0.15 g of LiAlH₄ (4.1 mmol) was added and the mixture was refluxed for 2 hours.
The mixture was then cooled to room temperature and quenched with 0.30 ml water, then washed with 0.30 ml of 15% aqeous NaOH and 0.60 ml water, then diluted with 15 ml of diethyl ether.The precipitate was removed by filtration.The organic phase was separated, dried over MgSO₄ and concentrated to give 0.7g (73%) of a yellow oil.

### Description 6

### 3-Chloro-N-(3,4-dichlorophenyl)propanamide.

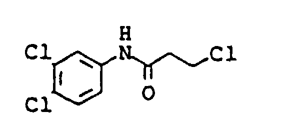

To a solution of 5.00 g (30 mmol) of 3,4-dichlorobenzeneamine and 3.30 g (38 mmol) of triethylamine in 30 ml of methylene chloride cooled to 0°C 3.81 (30 mmol) of 3-chloropropionyl chloride neat or dissolved in 5 ml methylene chloride were added dropwise and the reaction mixture was allowed to stir for 1 hour. The reaction mixture was then washed with 100 ml water and 100 ml of 0.5N aqueous HCl, then again with water until neutral.The organic phase was separated, dried over Na₂SO₄, concentrated and triturated with diisopropyl ether to afford 5.21 g (67%) of an off-white product.
mp = 126-127°C.

### Description 7

### N-(3,4-Dichlorophenyl)-3-[[2-(3,4-dimethoxyphenyl)]ethyl]methylamino]-propanamide.

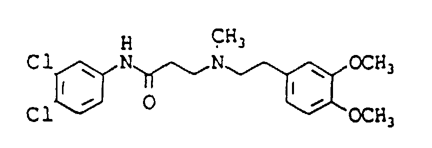

To a stirred solution of 5.2 g (20 mmol) of 3-chloro-N-(3,4-dichlorophenyl)propanamide (D6) and 1.74 g (20 mmol) of triethylamine in 50 ml of acetonitrile, 3.90 g ( 20 mmol) of 3,4-dimethoxy-N-methylbenzeneethanamine were added and the reaction mixture was refluxed for 24 hours.
On cooling, the reaction mixture was concentrated to dryness. 200 ml of ethyl acetate were added and the solution was washed with 100 ml water.
The organic phase was separated, dried over Na₂SO₄ and concentrated, to afford 4.32 g (90%) of a pale yellow oil which was used directly in a subsequent reaction.

### Description 8

### N-(3,4-Dichlorophenyl)-N'-[2-(3,4-dimethoxyphenyl)ethyl]-N'-methyl-1,3-propanediamine

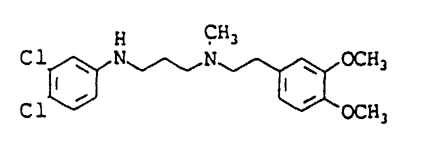

To a stirred solution of 4.32 g (11 mmol) of N-(3,4-dichlorophenyl)-3-[[2-(3,4-dimethoxyphenyl)ethyl]methylamino]-propanamide (D7) in 40 ml of tetrahydrofuran,0.59 g (16 mmol) of LiAlH₄ were added cautiously, and the reaction mixture was refluxed for 4 hours.The mixture was then cooled to room temperature and quenched with 0.56 ml water then washed with 0.56 ml of 15% aqueous NaOH, then with 1.03 ml of water The organic phase was diluted with 15 ml diethyl ether.The precipitate was removed by filtration.The organic phase was separated, dried over MgSO₄ and concentrated *in vacuo* to give a yellow oil. This was purified by flash chromatography on silica gel using 3:1 methylene chloride : ethyl acetate and then 9:1 methylene chloride : methanol to afford 2.6 g (62%) of a pale yellow oil.

### Description 9

### 3-Chloro-N-(3,4,5-trimethoxyphenyl)propanamide.

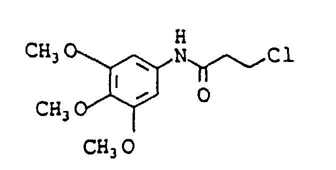

To an ice cooled stirred solution of 5.50 g (30 mmol) of 3,4,5 trimethoxybenzeneamine and 3.30 g (38 mmol) of triethylamine in 30 ml of methylene chloride, 3.81 g (30 mmol) of 3-chloropropionyl chloride neat or dissolved in 5 ml methylene chloride were added dropwise and the reaction mixture was allowed to stir for 1 hour.The reaction mixture was then washed with 100 ml of water, 100 ml of 0.5N aqueous HCl, and again with water until neutral. The organic phase was separated, dried over Na₂SO₄ concentrated and triturated with diisopropyl ether to afford 6.70 g (82%) of an off-white solid.
mp = 99-100°C.

### Description 10

### N-(3,4,5-Trimethoxyphenyl)-3-[[2-(3,4-dimethoxyphenyl)ethyl]methylamino]-propanamide

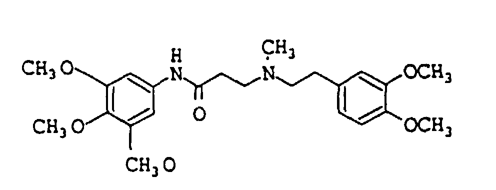

To a stirred solution of 6.70 g (20 mmol) of 3-chloro-N-(3,4,5-trimethoxyphenyl)propanamide (D9) and 1.74 g (20 mmol) of triethylamine in 50 ml of acetonitrile, 3.90 g (20 mmol) of 3,4-dimethoxy-N-methylbenzeneethanamine were added and the reaction mixture was refluxed for 24 hours.
On cooling, the reaction mixture was concentrated *in vacuo* to dryness, 200 ml of ethyl acetate were added and the solution was washed with 100 ml of water. The organic phase was separated, dried over Na₂SO₄ and concentrated affording 7.4 g (70%) of a pale yellow oil which was used directly in a subsequent reaction.

### Description 11

### N-(3,4,5-Trimethoxyphenyl)-N'-[2-(3,4-dimethoxyphenyl)ethyl]-N'-methyl-1,3-propanediamine

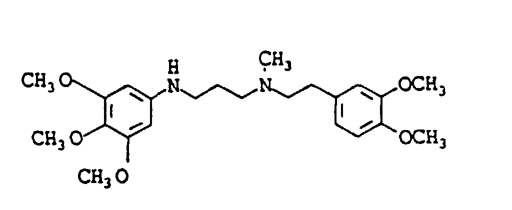

To a stirred solution of 7.4 g (17 mmol) of N-(3,4,5-trimethoxyphenyl)-3-[[2-(3,4-dimethoxyphenlyl)ethyl]methylamino]-propanamide (D10) in 50 ml of tetrahydrofuran, 0.97 g of LiAlH₄ (26 mmol) were added cautiously and the reaction mixture was refluxed for 4 hours.
The mixture was then cooled to room temperature and quenched with 1.03 ml water, then washed with 1.03 ml of 15% aqueous NaOH then with 2.6 ml of water. The organic phase was diluted with 15 ml of diethyl ether and the precipitate was removed by filtration.The organic phase was then separated, dried over MgSO₄ and concentrated *in vacuo* to give a pale pink oil. This was purified by flash chromatography on silica gel using 9:1 methylene chloride : methanol to afford 1.14 g (16%) of a pale pink oil.

### Description 12

### 3,4-Dimethoxy-N-(2-propenyl)-benzeneethanamine

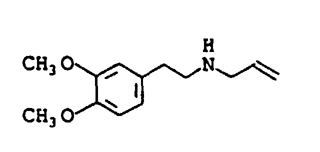

To a stirred solution of 5.43 g (30 mmol) of 3,4-dimethoxybenzeneethanamine in 60 ml acetonitrile, 5.14 g (30 mmol) of K₂CO₃ were added.Then 3.63 g (30 mmol) of allyl bromide in 20 ml acetonitrile were added dropwise over a period of 30 minutes. Agitation was maintained at room temperature overnight. The mixture was then concentrated *in vacuo* to dryness, dissolved in 100 ml of diethyl ether washed three times with 100 ml water, dried over MgSO₄ and concentrated *in vacuo*. Purification by flash chromatography on silica gel using 95:5 methylene chloride : methanol afforded 1.70 g (25%) of an oil.

### Description 13

### N-(3,4-Dimethoxyphenyl)-3-[[2-(3,4-dimethoxyphenyl)ethyl]2-propenylamino]-propanamide

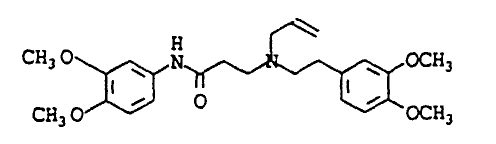

To a stirred solution of 1.62 g (7.3 mmol) of 3,4-dimethoxy-N-(2-propenyl)-benzeneethanamine (D12) and 0.73 g (7.3 mmol) of triethylamine in 20 ml of acetonitrile 1.80 g (7.3 mmol) of 3-chloro-N-(3,4-dimethoxyphenyl)-propanamide were added and the mixture was refluxed for 20 hours.
On cooling the mixture was concentrated to dryness *in vacuo*, the residue dissolved in 100 ml ethyl acetate, washed with 100 ml water and extracted with 100 ml of 1N aqueous HCl. The aqueous phase was separated, basified with 1N aqueous NaOH and the product was extracted twice with 50 ml ethyl acetate, washed twice with 50 ml of water, dried over MgSO₄ and concentrated *in vacuo*.
Purification by flash chromatography on silica gel using 95:5 ethyl acetate : ethanol followed by concentration to dryness yielded 1 g (32%) of an oil.

### Description 14

### N-(3,4-Dimethoxyphenyl)-N'-[2-(3,4-dimethoxyphenyl)ethyl]-N'-2-propenyl-1,3-propanediamine

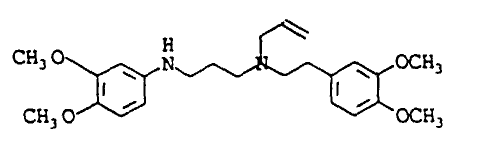

To a stirred solution of 1.0 g (2.4 mmol) of N-(3,4-dimethoxyphenyl)-3-[[2-(3,4-dimethoxyphenyl)ethyl]2-propenylamino]-propanamide (D13) in 15 ml of tetrahydrofuran, 0.133 g (3.5 mmol) of LiAlH₄ were added cautiously and the reaction mixture was then refluxed for 3 hours. After cooling to room temperature it was quenched with 0.15 ml water, then washed with 0.15 ml of 15% aqueous NaOH and 0.45 ml water.The organic phase was diluted with 15 ml of diethyl ether and the precipitate removed by filtration. The organic phase was then separated, washed twice with 20 ml water, dried over MgSO₄ and concentrated *in vacuo*.
Purification by flash chromatography on silica gel using 97:3 methylene chloride : ethanol afforded 0.25 g (25%) of an oil.

### Description 15

### 3-Chloro-N-3,4-bis(1-methylethoxy)phenyl-propanamide

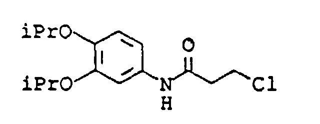

2.0 g (16 mmol) of 3-chloropropionyl chloride were added dropwise to an ice cooled stired solution of 3 g (14 mmol) of 3,4-bis(1-methylethoxy)benzamine and 1.59 g (16 mmol) of triethylamine in 30 ml methylene chloride. The mixture was stirred for 1 hour at room temperature and 20 ml water were added. The organic phase was washed sucessively with 20 ml 0.1N aqueous hydrochloric acid, water, a saturated aqueous NaHCO₃ solution, again with water and then dried over MgSO₄ and concentrated *in vacuo* to dryness. The residue was triturated in 75 ml of diisopropyl ether to yield 2.5 g (59.6%) of the desired compound as white crystals.m.p = 106°C
¹H NMR(CDCl₃) δ = 1.31 (d,6H,J=6.4Hz,CH₃x2); 1.34 (d,6H,J=6.4Hz,CH₃x2); 2.79 (t,2H,J=6.4Hz,CH₂-CO); 3.88 (t,2H,J=6.4Hz,CH₂Cl); 4.36-4.46 (m,2H,CH(CH₃)₂); 6.80-6.95 (broad band,2H,Ar); 7.21 (broad band,1H,exchD₂O,NH); 7.31 (s,1H,Ar) ppm.

### Description 16

### N-[3,4-bis(1-methylethoxy)phenyl]-3-[[2-(3,4-dimethoxyphenyl)ethyl]methylamino]propanamide

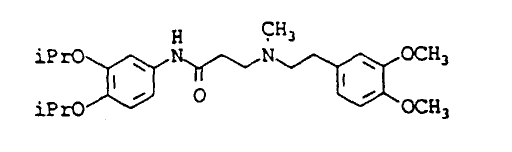

A solution of 1.95 g (10 mmol) of N-Methyl-3,4-dimethoxybenzenethanamine 3 g (10 mmol) of 3-chloro-N-3,4-bis (1-methylethoxy)phenyl-propanamide and 1.52 ml (11 mmol) of triethylamine in 50 ml acetonitrile was refluxed for 18 hours under stirring. The solvent was concentrated to dryness and the residue taken up in 50 ml methylene chloride. The organic solution was washed with water, dried over MgSO₄ and the solvent was evaporated *in vacuo*. The resulting brown oil was purified by chromatography on silica gel using 99:1 methylene chloride:methanol to afford 3.74 g (81.5%) of a light brown oil.
¹H NMR (CDCl₃) δ = 1.30 (d,6H,J=6.1Hz,CH₃x2); 1.33 (d,6H,J=6.1Hz,CH₃x2); 2.42 (s,3H,NCH₃); 3.81 and 3.84 (2s,6H,2CH₃O); 4.37 and 4.50(2 hept,2H,J=6.1Hz,2CH); 6.56(dd,2H,J=2.5Hz,J'=8.5Hz,Ar); 6.70-6.73(m,3H,,Ar); 6.81(d,1H,J4=8.5Hz,Ar); 7.40(d,1H,J=2.5Hz,Ar); 1O.53(s,1H,exch.D2O,NH)ppm.

### Description 17

### N-[2-(3,4-dimethoxyphenyl)ethyl]-N-methyl-N'-[3,4-bis(1-methylethoxy)phenyl]-1,3-propanediamine

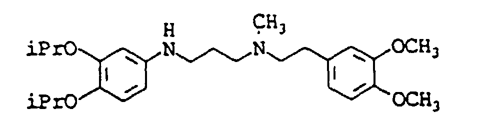

0.139g (3,6 mmol) of lithium aluminium hydride were added by small fractions, under stirring, to 10 ml dry tetrahydofuran (THF) cooled at 0°C. Then a solution of 1 g of (2.2 mmol) of N-[3,4-bis(1-methylethoxy) phenyl]-3-[[2-(3,4-dimethoxyphenyl)ethyl]methylamino]propanamide (D16) in 5 ml dry THF was added dropwise. The mixture was stirred some minutes at room temperature then refluxed for 3 hours. Then 0.14 ml of water were added dropwise followed by 0.14 ml of 15% aqueous NaOH and again 0.14 ml of water. The precipitate was filtered off, washed twice with 5 ml of ethyl acetate and the organic solutions were concentrated *in vacuo* to dryness. The residue was purified by chromatography on silica gel using first 98:2 then 95:5 methylene chloride:methanol to afford 817 mg (84.5%) of a brown oil.
¹H NMR (CDCl₃) δ = 1.27 and 1.32 (2d,12H,J=6.1Hz,(CH₃)ₓ₄); 1.82 (q,2H,J=6.6HzCH₂); 2.36 (s,3H,NCH₃); 2.53-2.84 (m,6H,3CH₂); 3.13 (t,2H,J=6.6Hz,CH₂); 3.85 and 3.87 (2s,6H,2CH₃O); 4.24 and 4.48 (2 hept,2H,J=6.1Hz,2CH(CH₃)₂); 6.13 (dd,1H,J=2.7Hz,J'=8.5Hz,Ar); 6.22 (d,1H,J=2.7Hz,Ar); 6.70-6.82 (m,4H,Ar) ppm.

### Description 18

### N-(3,4-Dimethoxyphenyl)-3-[[2-(3,4-dimethoxyphenyl)ethyl](1-methylethyl)amino] propanamide

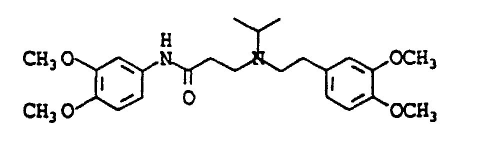

Starting from 2.0 g (8.9 mmol) of N-(1-methylethyl)-3,4-dimethoxy benzeneethanamine and 2.18 g (8.9 mmol) of 3-chloro-N-3,4-dimethoxyphenyl propanamide and following the method described in description 16, yielded 580 mg (15%) of the title compound as purple oil. The compound was purified by chromatography on silica gel using 95:5 methylene chloride: methanol.
¹H NMR (CDCl₃) δ = 1.11 (d,6H,J=6.6Hz,(CH₃)₂-CH); 2.45-2.60 (m,2H,CH₂CO); 2.75-2.90 (m,6H,3CH₂); 3.26 (hept,1H,J=6.64,NCH(CH₃)₂); 3.79, 3.83,3.85 and 3.87 (4s,12H,4CH₃O); 6.57-6.81 (m,5H,Ar); 7.44 (s,1H,Ar); 10.65 (s,1H,exch D₂O,NH)ppm.

### Description 19

### N-(3,4-Dimethoxyphenyl)-N'-[2-(3,4-dimethoxyphenyl)ethyl)-N'-(1-methylethyl)-1,3-propanediamine

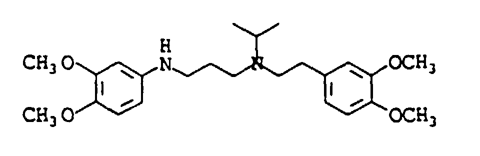

Starting from 580 mg (1.3 mmol) of N-(3,4-dimethoxyphenyl)-3-[[2-(3,4-dimethoxyphenyl) ethyl](1-methylethyl)amino]propanamide (D18) and following the method described in description 17, yielded 180 mg (33.3 %) of the title compound as yellow oil. The compound was purified by chromatography on silica gel using 98:2 methylene chloride:methanol.
¹H NMR (CDCl₃) δ = 1.11 (d,6H,J=5.7Hz,(CH₃)₂-CH); 1.78-2.00 (m,2H,CH₂); 2.67-2.92 (m,6H,3CH₂); 3.16 (t,2H,J=6.2Hz,CH₂); 3.00-3.20 (m,1H,CH(CH₃)₂); 3.80, 3.84, 3.85 and 3.86 (4s,12H,4CH₃)₂); 6.12 (dd,1H,J=2.6Hz,J'=8.5Hz,Ar); 6.23 (d,1H,J=2.6Hz,Ar); 6.70-6.82 (m,4H,Ar)ppm.

### Description 20

### 3-Chloro-N-(3,4-diethoxyphenyl)-propanamide

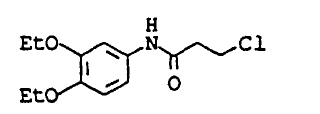

3.7 g (29 mmol) of 3-chloropropionyl chloride were added dropwise to an ice cooled stirred solution of 5.0 g (27.6 mmol) of 3,4-diethoxybenzeneamine and 3.07 g (30 mmol) of triethylamine in 40 ml methylene chloride. The mixture was stirred for 15 hours at room temperature, then washed with water, with 0.1N aqueous HCl, with a saturated aqueous solution of NaHCO₃ and again with water. The organic solution was dried over MgSO₄. Some silica gel was added, stirred some minutes, and filtered off to remove polar impurities. The solvent was concentrated to dryness *in vacuo* and the residue was triturated in diisopropyl ether to afford 4.8 g (63.7%) of crystals.
m.p = 118°C
¹H NMR (CDCl₃) δ = 1.36-1.51 (m.6H,2CH₃); 2.79 (t,2H,J=6.4Hz,CH₂CO); 3.88 (t,2H,J=6.4Hz,CH₂Cl); 4.00-4.17 (m,4H,2CH₂); 6.77-6.92 (m,2H,Ar); 7.32 (s,1H,Ar); 7.28-7.40 (s,1H,exch D₂O,NH)ppm.

### Description 21

### N-(3,4-Diethoxyphenyl)-3-[[2-(3,4-diethoxyphenyl)ethyl]methylamino]propanamide

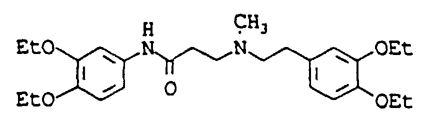

Starting from 1.47 g (6.6 mmol) of N-methyl-3,4-diethoxybenzeneethanamine and 1.8 g (6.6 mmol) of 3-chloro-N-3,4-diethoxyphenyl)-propanamide (D20) and following the method described in description 16, yielded 1.65 g (54.5 %) of the title compound as brown oil. The compound was purified by chromatography on silica gel using 95:5 methylene chloride:methanol.
¹H NMR (DMSO-d₆) δ = 1.23-1.37 (m,12H,4CH₃); 2.24 (s,3H,CH₃n); 2.40 (t,2H,J=6.6Hz,CH₂); 2.50-2.77 (m,6H,3CH₂); 3.39-4.03 (m,8H,4CH₂O); 6.69 (dd,1H, J=1.5Hz,J'=8.1Hz,Ar); 6.73-6.89 (m,3H,Ar); 6.99 (dd,1H,J=2.2Hz,J'=8.7Hz,Ar); 7.29 (d,1H,J=2.2Hz,Ar); 9.85 (s, 1H,exch D₂O,NH)ppm.

### Description 22

### N-(3,4-Diethoxyphenyl)-N'-[2-(3,4-diethoxyphenyl)ethyl]-N'-methyl-1,3-propanediamine

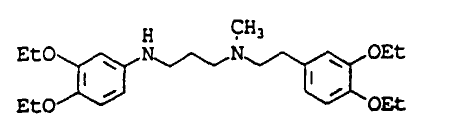

Starting from 1.65 (3.6 mmol) of N-(3,4-Diethoxyphenyl)-3-[[2-(3,4-diethoxyphenyl)ethyl] methylamino]propanamide (D21) and following the method described in description 17, yielded 1.1 g (68.7 g) of the title compound as brown oil. The compound was purified twice by chromatography on silica gel using first 92.5:7.5 methylene:methanol then 90:10 ethylacetate:methanol.
¹H NMR (DMSO-d₆) δ = 1.17-1.36 (m,12H,4CH₃); 1.65 (q,2H,J=6.7Hz,CH₂); 2.22 (s,3H,CH₃N); 2.38-2.68 (m,6H,3CH₂); 2.93 (t,2H,J=6.7Hz,CH₂); 3.77-4.03 (m,8H,4CH₂O); 5.15 (s,1H,exch D2O,NH); 5.99 (dd,1H,J=2.2Hz,Ar);6.53-6.74 (m,4H,Ar) ppm.

### Description 23

### N-Cyclopropyl-3,4-dimethoxybenzeneacetamide

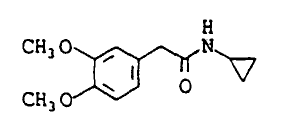

26.4 g (0.123 mol) of 3,4-dimethoxybenzeneacetyl chloride were added dropwise to a stirred solution of 6.7 g (0.12 mol) of cyclopropanamine and 13.35 g (0.132 mol) of triethylamine in 200 ml methylene chloride. The reaction mixture was stirred for 15h at room temperature then washed twice with water, dried over MgSO₄ and concentrated *in vacuo* to dryness affording 25.2 g (89.4%) of yellow crystals.
¹H NMR (DMSO-d₆) δ = 0.32-0.42 (m,2H,CH₂-CH₂); 0.53-0.64 (m,2H,CH₂-CH₂); 2.59 (m,H,CH); 3.25 (s,2H,CH₂CO); 3.71 and3.72 (2s,6H,2CH₃O); 6.73 (dd,1H,J=1.9Hz, J'=8.1Hz,Ar); 6.84 (s,1H,Ar); 6.86 (d,1H,J'=8.1Hz,Ar); 8.05 (s,1H,exch D₂O,NH) ppm.

### Description 24

### N-Cyclopropyl 3,4-dimethoxybenzeneethanamine

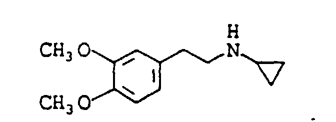

A solution of 12 ml of acetic acid in 15 ml of dioxane was added dropwise to a stirred solution of 10 g (42 mmol) of N-cyclopropyl-3,4-dimethoxybenzeneacetamide (D23) and 8.04 g (210 mmol) of sodium borohydride in 50 ml of dioxane, at room temperature. The reaction mixture was refluxed for 3 hours under stirring then poured onto 500 g of crushed ice. The aqueous phase was extracted four times with 150 ml of methylene chloride and the organic solution was extracted three times with 2N aqueous HCl. The acidic phases were washed with 100 ml ethylacetate, basified with 35% aqueous NaOH and extracted three time with 150 ml of methylene chloride. The organic solution was dried over MgSO₄ and concentrated *in vacuo* to dryness affording 5.5 g (59%) of a yellow oil.
¹H NMR (DMSO-d₆) δ = 0.12-0.23 (m,2H,CH₂-CH₂); 0.33-0.40 (m,2H,CH₂-CH₂); 1.98-2.14 (m,2H,CH and NH); 2.63 (t,2H,J=6.7Hz,CH₂ Ar); 2.78 (t,2H,J=6.7Hz,CH₂N); 3.70 and 3.73 (2s,6H,2CH₃O); 6.15-6.87 (m,3H,Ar) ppm.

### Description 25

### N-(3,4-Dimethoxyphenyl)-3-[[2-(3,4-dimethoxyphenyl)ethyl]cyclopropylamino]propanamide

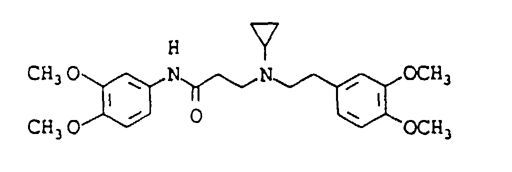

A solution of 2.5 g (11.2 mmol) of N-cyclopropyl 3,4-dimethoxybenzeneethanamine (D23), 5.5 g (22.5 mmol) 3-chloro-N-3,4-dimethoxyphenyl propanamide and 3.4 ml (22.5 mmol) triethylamine in 50 ml acetonitrile were refluxed for 15 hours under stirring. The reaction mixture was poured on water and extracted with methylene chloride. The methylene chlordie solution was washed with water, dried over MgSO₄ and concentrated to dryness. The residue was chromatographed on silica gel using ethyl acetate then 95/5 ethyl acetate/methanol. The compound was then taken up in methylene chloride and extracted with 2N aqueous HCl. The acid aqueous phase was basified with 30% aqueous NaOH and extracted with ethyl acetate. The organic solution was dried over MgSO₄ and concentrated *in vacuo* to dryness affording 1.42 g (29.6%) of the title compound as brown oil.
¹H NMR (DMSO-d₆) δ = 0.24-0.53 (m,4H,(CH₂)₂); 1.80-1.93 (m,1H,CH) ; 2.57-2.88 (m,4H,2CH₂); 2.88-3.03 (m,2H,CH₂); 3.63-3.76 (m,14H,4CH₃O and CH₂); 6.71 (dd,1H,J=1.6Hz,J'=8.2Hz,Ar); 6.76-6.90 (m,3H,Ar); 7.06 (dd,1H,J=2.2Hz,J'=8.2Hz,Ar); 7.28 (d,1H,J=2.2Hz,Ar); 9.80 (s,1H,exch D₂O,NH) ppm

### Description 26

### N-(3,4-Dimethoxyphenyl)-N'-[2-(3,4-dimethoxyphenyl)ethyl]-N'-cyclopropyl-1,3-propanediamine

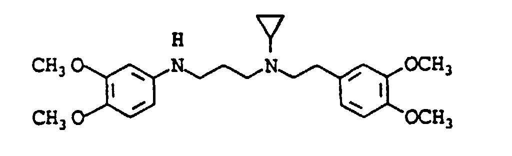

Starting from 1.37 g (3.2 mmol) N-(3,4-Dimethoxyphenyl)-N-3-[[2-(3,4-dimethoxyphenyl) ethyl]cyclopropylamino]propynamide (D25) and following the method described in Description 17, yielded 0.78 g (58.7%) of the title compound as brown oil. The compound was purified by chromatography on silica gel using 95/5 methylene chloride/methanol.
¹H NMR (DMSO-d₆) δ = 0.23-0.53 (m,4H,(CH₂)₂); 1.53-1.88 (m,3H,CH₂ and CH) ; 2.60-2.83 (m,6H,3CH₂); 2.87-3.00 (m,2H,CH₂); 3.61, 3.68, 3.70 and 3.72 (4s,12H,4CH₃O); 5.09-5.23 (broad band, 1H,exch D₂O,NH); 6.01 (dd,1H,J=2.3Hz,J'=8.6Hz,Ar); 6.24 (d,1H,J=2.3Hz,Ar); 6.65-6.87 (m,4H,Ar) ppm

### Description 27

### 3-Chloro-N-[3-methoxy-4-(phenylmethoxy)phenyl]propanamide

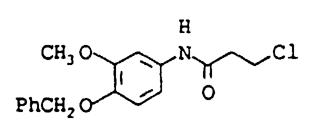

Starting from 3.3 g (0.014 mole) [3-methoxy-4-(phenylmethoxy)]benzeneamine and following the method described in Description 15, yielded 3.19 g (69.3%) of the title compound as pink crystals. The compound was purified by chromatography on silica gel using 98/2 methylene chloride/methanol.
m.p = 121°C
NMR (CDCl₃) δ = 2.78(t,2H,J=6.4Hz,CH₂CO); 3.87 (t,2H,J=6.4Hz,CH₂Cl); 3.88 (s,3H,CH₃O); 5.12 (s,2H,OCH₂Ar); 6.75-6.84 (m,2H,Ar); 7.27-7.48 (m,6H,Ar) ppm

### Description 28

### 3-[[2-(3,4-Dimethoxyphenyl)ethyl]methylamino]-N-[3-methoxy-4-(phenylmethoxy)phenyl]-propanamide

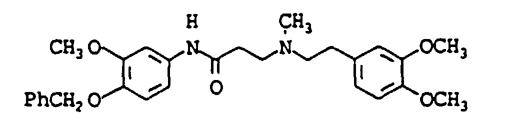

A soluton of 3 g (9.4 mmol) of 3-chloro-N-[3-methoxy-4-(phenylmethoxy)phenyl]propanamide (D27), 1.83 g (9.4 mmol) N-methyl-3,4-dimethoxybenzeneethanamine and 1.04 g (10.3 mmol) triethylamine in 50 ml acetonitrile were refluxed for 4 hours. The solvent was concentrated to dryness and the residue taken up in 50 ml methylene chloride. The organic solution was washed with water, dried over MgSO₄ and the solvent was evaporated *in vacuo*. The resulting pink oil was chromatographed on silica gel using 98.5/1.5 methylene chloride/methanol affording 3.47 g (77.4%) of the title compound as light pink crystals.
m.p = 121°C
¹H NMR (DMSO-d₆) δ = 2.25 (s,3H,NCH₃); 2.36-2.78 (m,8H,4CH₂); 3.68, 3.70 and 3.73 (3s,9H,3CH₃O); 5.02 (s,2H,CH₂); 6.67-7.04 (m,5H,Ar); 7.28-7.47 (m,6H,Ar) ; 9.90 (s,1H,exch D₂O,NH) ppm

### Description 29

### N-[(3-Methoxy-4-(phenylmethoxy)phenyl]-N'-[2-(3,4-dimethoxyphenyl)ethyl]-N'-methyl-1,3-propanediamine.

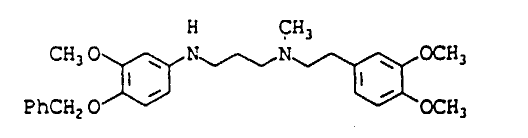

Starting from 3 g (6.3 mmol) 3-[[2-(3,4-dimethoxyphenyl)ethyl]-methylamino]-N-[3-methoxy-4-(phenylmethoxy)phenyl]propanamide (D28) and following the method described in Description 17, yielded 669 mg (22.9%) of the title compound as brown oil. The compound was purified by chromatography on silica gel using 98/2 methylene chloride/methanol.
¹H NMR (DMSO-d₆) δ = 1.66 (q,2H,J=6.7Hz,CH₂); 2.23 (s,3H,CH₃NH), 2.45-2.74 (m,6H,3CH₂); 2.95 (t,2H,J=6.7Hz,CH₂); 3.69, 3.71 and 3.72 (3s,9H,3CH₃O); 4.89 (s,2H,OCH₂Ar) ; 5.97 (dd,1H,J=2.3Hz,J'=8.5Hz,Ar); 6.25 (d,1H,J=2.3Hz,Ar); 6.65-6.85 (m,4H,,Ar); 7.38-7.46 (m,5H,Ar) ppm

### Description 30

### N-(4-Hydroxy-3-methoxyphenyl)-N'-[2-(3,4-dimethoxyphenyl)ethyl]-N'-methyl-1,3-propanediamine

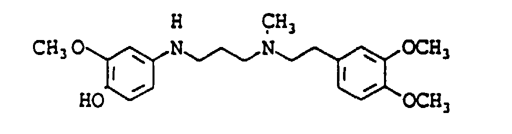

1.06 g (2.3 mmol) N-[2-(3,4-Dimethoxyphenyl)ethyl]-N-methyl-N'-[(3-methoxy-4-phenylmethoxy)phenyl]-1.3--propanediamine (D20) and 0.12 g 10% Pd/C were hydrogenated in 50 ml methanol at 15°C under 5 to 10 bars for 4 hours. The catalyst was filtered off and the solvent concentrated to dryness. The resulting brown oil was chromatographed on silica gel using 98/2 methylene chloride/methane affording 510 mg (54%) of the title compound as brown oil.
¹H NMR (DMSO-d₆) δ = 1.66 (q,2H,J=6.8Hz,CH₂), 2.23 (s,3H,NCH₃); 2.37-2.72 (m,6H,3CH₂); 2.93 (t,2H,J=6.6Hz,CH₂); 3.68, 3.70 and 3.72 (3s,9H,3CH₃O); 4.35 (broad band,1H,exch D₂O,ArOH) 5.94 (dd,1HJ=2.3Hz,J'=8.3Hz,Ar); 6.20 (d,1H,J=2.3Hz,Ar); 6.52 (d,1H,J=8.3Hz,Ar); 6.65-6.86 (m,3H,Ar); 7.85 (s,1H, exch D₂O,NH) ppm

### Example 1

### N-[2-(3,4-Dimethoxyphenyl)ethyl]-N'-[3-[[2-(3,4-dimethoxyphenyl)ethyl]methylamino]propyl] -4-nitrobenzamide hydrochloride

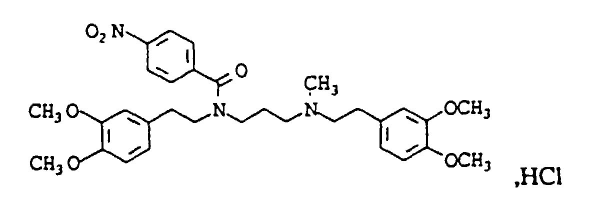

A solution of 6.4 g, (15 mmol) of N-[2-(3,4-dimethoxyphenyl)ethyl]-3-[2-(3,4-dimethoxyphenyl)ethyl]methylamino]-propanamide in 100 ml of THF was added dropwise under argon to a suspension of 1.13 g (30 mmol) LiALH₄ in 100 ml of THF. The reaction mixture was then refluxed for two and half hours. After cooling to room temperature, the reaction was quenched by 1 ml of water, then washed by 1 ml of 15% aqueous NaOH, and 3 ml of water. The inorganic solid was removed by filtration and washed with THF. The filtrate was concentrated *in vacuo*. The resulting crude product was dissolved in diethyl ether, washed with brine, dried over MgSO₄, and concentrated *in vacuo* to dryness, affording 5 g of a yellow oil. A mixture containing 5 g, (12 mmol) of the isolated oil, 2.5 g (13 mmol) of 4-nitrobenzoyl chloride, and 1.4 g (14 mmol) of triethylamine in 150 ml of chloroform was refluxed under argon for 9 hours. After cooling down to room temperature, the reaction mixture was washed with water. The organic layer was dried over MgSO₄ and concentrated *in vacuo* to dryness. The resulting crude product was purified by flash chromatography on silica gel using 95:5 methylene chloride:methanol, affording 1 g (12%) of an amorphous solid.The product was then disolved in 5 ml of ethyl acetate and 5.5N anhydrous HCl in diethyl ether was added dropwise. The resulting amorphous solid was triturated in anhydrous diethyl ether and afforded 0.9 g (85%) of the title compound as yellow cristals.
m.p = decomp.
¹H NMR (DMSO-d₆) : presence of syn and anti forms (ratio = 75/25) δ = 1.85-2.25 (m,2H,CH₂); 2.50-3.50 (m,13H,(CH₂)x5,NCH₃); 3.50-3.85 (m,14H, (OCH₃)x4,CH₂); 6.40-7.00 (m,6H,Ar); 7.30-7.65 (m,2H,Ar); 8.10-8.35 (m,2H,Ar); 10.45-10.75 (m, 1H,exch.D₂O,NH) ppm.

### Example 2

### N-(4-Fluorophenyl)-N'-[3-[[2-(3,4-dimethoxyphenyl)ethyl] methylamino]propyl]-4-nitrobenzamide

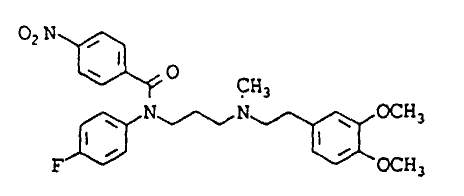

A solution of 0.7 g (2.0 mmol) of N'-(4-fluorophenyl)-N-[-2-(3,4-dimethoxyphenyl)ethyl]-N-methyl-1,3-propanediamine (D5), and 0.41 g (4.0 mmol) of triethylamine in 30 ml of chloroform was stirred in an ice bath.Then 0.45 g (2.4 mmol) of 4 nitrobenzoyl chloride dissolved in 10 ml chloroform were added dropwise.Agitation was maintained for 1 hour.
100 ml ethyl acetate were added and the mixture was washed twice with 100 ml of water, then with 50 ml of 1N aqueous HCl. The aqueous phase was basified with NaOH and the product was extracted with ethyl acetate.The organic phase was then separated, dried over MgSO₄ and concentrated *in vacuo* resulting in an orange oil.This last was purified by flash chromatography on silical gel using 9:1 methylene chloride : methanol. The product was then dissolved in diethyl ether and 0.1 ml of 5.5N anhydrous HCl in ether was added. The mixture was concentrated and dried *in vacuo* to afford 0.4 g (69%) of a yellow mousse. m.p = 80° C.
¹H NMR(DMSO-d6) δ = 1.97 (t,2H,J=6.9Hz,CH₂); 2.78 (s,3H,NCH₃); 2.94 (t,2H,J=7.9Hz, CH₂); 3.24 (m,4H,CH₂x2); 3.72 (s,3Hx2,OCH₃x2); 3.92 (m,2H,CH₂NCO); 6.78 (d,1H,J=8.4Hz,Ar); 6.89-7.41 (m,6H,Ar); 7.54 (d,2H,J=8.4Hz,Ar)8.09 (d,2H,J=8.4Hz,AR); 10.40 (s,1H,exchD₂O,NH).

### Example 3

### N-(3,4-dichlorophenyl)-N-[3-[[2-(3,4-dimethoxyphenyl)ethyl] methylamino]propyl] -4-nitrobenzamide hydrochloride.

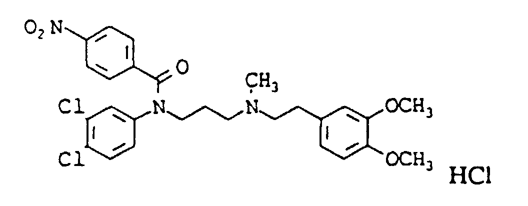

To an ice cooled stirred solution of 1 g (2.5 mmol) of N-(3,4-dichlorophenyl)-N'-[2-(3,4-dimethoxyphenyl)ethyl]-N'-methyl-1,3 propanediamine (D8) and 0.51 g (5 mmol) of triethylamine in 50 ml of chloroform, 0.56 g (3.0 mmol) of 4-nitrobenzoyl chloride dissolved in 10 ml of chloroform, were added dropwise. Agitation was maintained for 1 hour.
100 ml of ethyl acetate were added and the mixture was washed twice with 100 ml water, then with 50 ml of 1N aqueous HCl. The aqueous phase was then separated and basified with aqueous NaOH. 50 ml of ethyl acetate were added and the product was extracted. The organic phase was then separated, washed with water until neutral, dried over MgSO₄ and concentrated, resulting in a yellow oil. This was purified by flash chromatography on silica gel using 98:2 ethyl acetate : methanol. The product was then dissolved in 10 ml diethyl ether and 0.1 ml of 5.5M anhydrous HCl in ether were added. The mixture was concentrated and dried *in vacuo* at 60°C and 0.1 mmHg affording 1.2Og (87%) of a pale yellow mousse.
mp = 101°C.
¹H NMR(DMSO-d₆) δ = 1.96 (m,2H,CH2); 2.78 (s,3H,NCH3); 2.93 (m,2H,CH2); 3.23 (m, 4H, CH2x2); 3.72 (s,3H,OCH3); 3.75 (s,3H,OCH3); 3.95 (m,2H,CH2NCO); 6.77 (d,1H,J=8.3Hz,Ar); 6.90 (m,2H,Ar); 7.32 (m,1H,Ar); 7.58 (m, 3H,Ar); 7.79 (d,1H,j=2Hz,Ar); 8.15 (d,2H,J=8.3Hz,Ar); 10.37 (s, 1H,exch.D₂O,NH)ppm.

### Example 4

### N-(3,4,5-Trimethoxyphenyl)-N-[3-[[2-(3,4-dimethoxyphenyl)ethyl] methylamino]propyl]-4-nitrobenzamide hydrochloride.

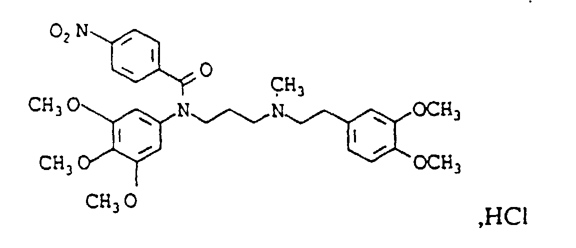

To an ice cooled stirred solution of 1 g (2.4 mmol) of N-(3,4,5-trimethoxyphenyl)-N'-[2-(3,4-dimethoxyphenyl)ethyl]-N'-methyl-1,3 propanediamine (D11) and 0.48 g (4.8 mmol) of triethylamine in 50 ml of chloroform, 0.53 g (2.9 mmol) of 4-nitrobenzoyl chloride dissolved in 10 ml chloroform were added dropwise. Agitation was maintained for 1 hour.Then 100 ml of ethyl acetate were added and the mixture was washed twice with 100 ml water, then extracted with 50 ml of 1N aqueous HCl. The aqueous phase was then separated and basified with NaOH. Then 50 ml of ethyl acetate were added and the product extracted. The organic phase was then separated, whashed with water until neutral, dried over MgSO₄ and concentrated. This was purified by flash chromatography on silica gel using 9:1 ethyl acetate : methanol. The product was then dissolved in 10 ml diethyl ether and 0.1 ml of 5.5M anhydrous HCl in ether were added. The mixture was concentrated and dried *in vacuo* at 60°C under 0.1 mm Hg to afford O.91g (63%) of a pale yellow foam. mp = 94°C.
¹H NMR(DMSO-d6) δ = 2.02 (m,2H,CH₂); 2.79 (s,3H,CH₃N); 2.96 (t,2H,CH₂); 3.25 (m,4H,CH₂x2), 3.56-3.74 (m,15H,OCH₃x5); 3.96 (m,2H,CH₂) 6.65 (s,2H,AR); 6.77 (d,1H,J=8.2Hz,Ar); 6.89 (m,2H,Ar); 7.61 (d,2H,J=8.4Hz,Ar); 8.10 (d,2H,J=8.4Hz,Ar); 10.70 (s.1H.exch.D2O,NH)ppm.

### Example 5

### N-(3,4-dimethoxyphenyl)-N-[3-[[2-(3,4-dimethoxyphenyl)ethyl]-2-propenylamino]propyl]-4-nitrobenzamide, hydrochloride.

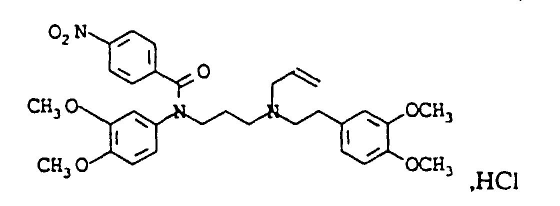

To a stirred solution of 0.25 g (0.6 mmol) of N-(3,4-dimethoxyphenyl)-N'-[2-(3,4-dimethoxyphenyl)ethyl]-N'-propenyl-1,3-propanediamine (D14) and 0.06 g (0.6 mmol) of triethylamine in 20 ml of methylene chloride 0.11 g (0.6 mmol) of 4-nitrobenzoyl chloride were added over a period of 10 minutes at room temperature. Agitation was maintained for 1 hour. Additional 20 ml of methylene chloride were added and the organic phase was washed three times with 30 ml water, dried over MgSO₄ and concentrated. Purification by flash chromatography on silica gel using ethyl acetate yielded 0.2 g (59%) of product
This product was dissolved in 10 ml ethyl acetate and 0.1 ml of 5.5M anhydrous HCl in diethyl ether was added. The mixture was concentrated and dried *in vacuo* at 60°C under 0.1 mmHg to yield 0.2g (54%) of the title compound as yellow solid. m.p. around 120°C
¹H NMR(DMSO-D6) δ = 2.02 (m,2H,CH₂); 2.93 (m,2H,CH₂); 3.21 (s,4H,CH₂x2); 3.66-3.74 (m,12H,OCH₃x4); 3.89 (m,4H,CH₂x2); 5.56 (m,2H,CH₂); 6.05 (m,1H,CH=CH₂); 6.76-6.96 (m,6H,Ar); 7.56 (d,2H,J=8.6Hz,Ar); 8.09 (d,2H,J+8.6Hz,Ar); 10.67 (s,1H,exchD2O,NH)ppm.

### Example 6

### 4-Amino-N-(3,4-dimethoxyphenyl)-N-[3-[[2-(3,4-dimethoxyphenyl)ethyl]methylamino] propyl]benzamide, dihydrochloride.

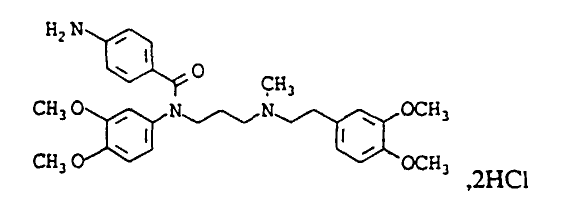

A mixture of 1.12 g (2.0 mmol) of N-(3,4-dimethoxyphenyl)-N-[3-[[2-(3,4-dimethoxyphenyl) ethyl]methylamino]propyl]-4-nitrobenzamide hydrochloride (described in, EP 233 762) and 2.25 g (10 mmol) of stannous chloride dihydrate in 15 ml ethanol were refluxed under stirring for 30 minutes. The solvent was concentrated to dryness and the residue taken up in water. The aqueous mixture was basified with an aqueous solution of NaHCO₃ and extracted three times with ethyl acetate. The organic solution was separated, dried over MgSO₄ and concentrated *in vacuo* to dryness. The crude base (0.88 g) was taken up in methanol and a solution of 5.5N anhydrous HCl in diethyl ether was added. The main part of the solvent was removed under reduced pressure and anhydrous diethyl ether was added to precipitate the desired dihydrochloride : 0.89 g (77%). m.p = 135-40°C
¹H NMR(DMSO-d₆) δ = 1.82-2.04 (m,2H,CH₂); 2.78 (d,3H,J=4.3Hz,CH₃NH); 2.86-2.99 (m,2H,CH₂); 3.04-3.37 (m,4H,2CH₂); 3.66, 3.69, 3.72 and 3.75 (4s,12H,4CH₃O); 3.78-3.94 (m,2H,CH₂); 6.60-6.93(m,8H,Ar); 7.16 (d,2H,J=8.4Hz,Ar); 10.36 (s,1H,exchD₂O,NH⁺) ppm

### Example 7

### N-[3-[[2-(3,4-Dimethoxyphenyl)ethyl]methylamino]propyl]-N-[3,4-bis(1-methylethoxy) phenyl]-4-nitrobenzamide,hydrochloride

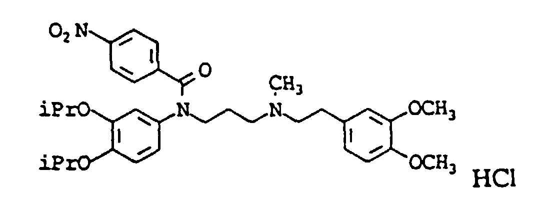

Starting from 800 mg (1.8 mmol) of N-[2-(3,4-dimethoxyphenyl)ethyl]-N-methyl-N'-[3,4-bis(1-methylethoxy)phenyl]1,3-propanediamine (D17) and 400 mg (2.16 mmol) of 4-nitrobenzoyl chloride and following the method described in example 4, 668 mg (58.9%) of the title compound were obtained as yellow crystals. The compound was purified as free base by chromatography on silica gel using 99:1 methylene chloride:methanol, and as hydrochloride by trituration first in diethylether then in diisopropyl ether.
m.p = 78°C
¹H NMR (DMSO-d₆) δ = 1.00-1.18 (m,12H,2x(CH₃)₂CH); 1.87-2.08 (m,2H,CH₂); 2.81 (d,3H,CH₃NH⁺); 2.85-3.02 (m,4H,CH₂); 3.08-3.40 (m,4H,2CH₂); 3.83-3.98 (m,2H,CH₂); 4.26-4.50 (m,2H,2xCH(CH₃)₂); 6.72-6.94 (m;6H,Ar); 7.55 (d,2H,J=8.5Hz,Ar); 8.08 (d,2H,J=8.5Hz,Ar); 10.12 (s,1H,exch D₂O,NH⁺) ppm.

### Example 8

### N-(3,4-Dimethoxyphenyl)-N-[3-[[2-(3,4-dimethoxyphenyl)ethyl](1-methylethyl)amino]propyl]-4-nitrobenzamide, hydrochloride

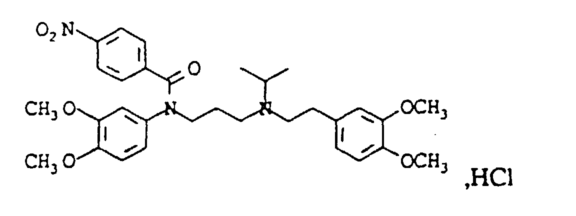

Starting from 540 mg (1.3 mmol) of N-(3,4-dimethoxyphenyl)-N'-[2-(3,4-dimethoxyphenyl) ethyl]-N'-(1-methylethyl)-1,3-propanediamine (D19) and 270 mg (1.4 mmol) of 4-nitrobenzoyl chloride and following the method described in example 4, yielded 420 mg (53.7%) of the title compound as a yellow solid. The compound was purified as free base by chromatography on silica gel using 95:5 methylene chloride:methanol and as hydrochloride by trituration in diethylether.
m.p = 105°C
¹H NMR (DMSO-d₆) δ = 1.18-1.35 (broad band,6H,(CH₃)₂CH); 1.95-2.20 (m,2H,CH₂); 2.85-3.05 (m,2H,CH₂); 3.05-3.32 (m,4H,2CH₂);3.28-3.43(m,1H,CH); 3.66 (s,6H,2CH₃O); 3.72 and 3.75 (2s,6H,2CH₃O); 3.85-4.02 (m,2H,CH₂); 6.75-6.98 (m;6H,Ar); 7.57 (d,2H,J=8.4Hz,Ar); 8.08 (d,2H,J=8.4Hz,Ar); 10.19 (s,1H,exch D₂O,NH⁺) ppm.

### Example 9

### N-(3,4-Diethoxyphenyl)-N-[3-[[2-(3,4-Diethoxyphenyl)ethyl]methylamino]propyl]-4-nitrobenzenecarboxamide, hydrochloride

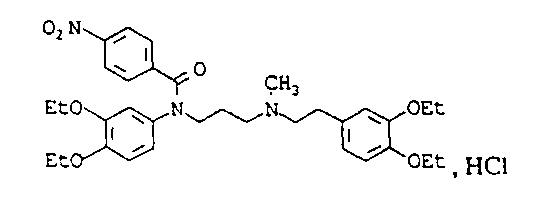

Starting from 1.04 g (2.3 mmol) of N-(3,4-Diethoxyphenyl)-N'-[2-(3,4-diethoxyphenyl)ethyl]-N'-methyl-1,3-propanadiamine (D22) and 486 mg (2.6 mmol) of 4-nitrobenzoylchloride and following the method described in example 4, yielded 1.05 g (72.5%) of the title compound as yellow solid. The compound was purified as free base by chromatography on silica gel using 95:5 methylene chloride:methanol.
¹H NMR (DMSO-d₆) δ = 1.12-1.35 (m,12H,4CH₃CH₂O); 1.87-2.05 (m,2H,CH₂); 2.78 (s,3H,CH₃); 2.82-3.00 (m,2H,CH₂); 3.08-3.38 (m,4H,2CH₂); 3.85-4.05 (m,10H,4CH₂O and CH₂N); 6.70-6.95 (m,6H,Ar); 7.56 (d,2H,J=8.5Hz,Ar); 8.08 (d2H,J=8.5Hz,Ar); 10.42 (s,1H,exch D₂O,NH⁺) ppm.

### Example 10

### N-(3,4-Dimethoxyphenyl)-N-[3-[[2-(3,4-dimethoxyphenyl)ethyl]methylamino]propyl]-N-(4-nitrophenyl)urea, hydrochloride

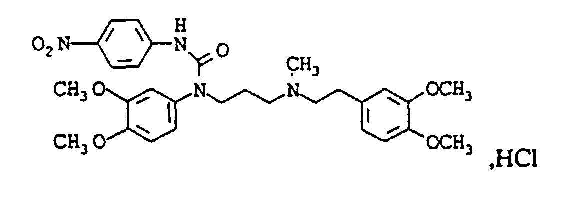

A solution of 1 g (2.6 mmol) of N-(3,4-dimethoxyphenyl)-N'-[2-(3,4-dimethoxyphenyl)ethyl]-N'-methyl-1,3-propanediamine (Int 1 prepared according to methods in EP 0233762) and 0.48 g (3 mmol) of 4-nitrobenzeneisocyanate in 25 ml dry methylene chloride was stirred at room temperature for 15 hours. The precipitate was filtered and purified by chromatography on silica gel using 95:5 methylene chloride:methanol yielding 0.83 g of a yellow solid which was salified in methanol by addition of 5.5N anhydrous HCl in diethyl ether. The crude salt was triturated in diethyl ether to afford 0.8 g (55.7%) of a yellow solid. m.p around 115°C.
¹H NMR (DMSO-d₆) δ = 1.80-1.98 (m,2H,CH₂); 2.78 (d,3H,J=4.5Hz,CH₃NH⁺); 2.86-3.02 (m,2H,CH₂); 3.08-3.43 (m,4H,2CH₂); 3.68-3.87 (m,2H,CH₂); 3.72, 3.74, 3.77 and 3.79 (4s,12H,4CH₃O); 6.72-7.07 (m,6H,Ar); 7.76 (d,2H,J=9.3Hz,Ar); 8.12 (d2H,J=9.3Hz,Ar); 8.48(s,1H,exch.D₂O,NH); 10.27(broad s,1H,exch.D₂O,NH+)ppm.

### Example 11

### N-(3,4-Dimethoxyphenyl)-N-[3[[2-(3,4-dimethoxyphenyl)ethyl]methylamino] propyl]benzamide, hydrochloride

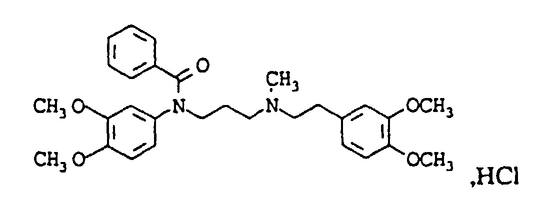

Starting from 800 mg (1.6 mmol) N-(3,4-dimethoxyphenyl)-N-[3[[2-(3,4-dimethoxyphenyl)ethyl]methylamino]propyl]benzamide and following the method of salification described in example 4, yielded 5.34 mg (63%) of the title compound as grey crystals. m.p = 75°C
¹H NMR (DMSO-d₆) δ = 1.83-2.05 (m,2H,CH₂); 2.80 (d,3H,J=4Hz,CH₃NH⁺); 2.88-3.00 (m,2H,CH₂); 3.05-3.37 (m,4H,2CH₂); 3.63, 3.73 and 3.75 (4s,12H,4CH₃O); 3.82-3.96 (m,2H,CH₂NCO); 6.62-6.93 (m,6H,Ar); 7.17-7.33 (m;5H,Ar); 10.25 (broad band,1H,exch D₂O,NH⁺) ppm.

### Example 12

### 4-Cyano-N-(3,4-Dimethoxyphenyl)-N-[3[[2-(3,4-dimethoxyphenyl)ethyl]methylamino]propyl] benzamide, hydrochloride

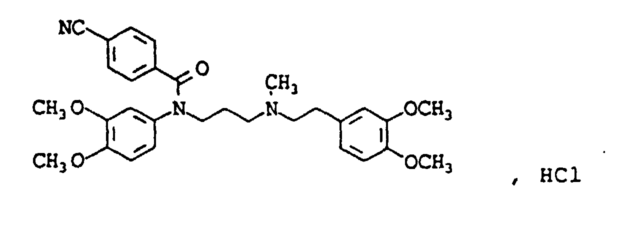

Starting from 1.26 mg (2.43 mmol) 4-Cyano-N-(3,4-dimethoxyphenyl)-N-[3[[2-(3,4-dimethoxyphenyl)ethyl]methylamino]propyl-benzamide and following the method of salification described in example 4, yielded 844 mg (62.7%) of the title compound as grey crystals. m.p = 90°C
¹H NMR (DMSO-d₆) δ = 1.83-2.10 (m,2H,CH₂); 2.79 (d,3H,J=4.1Hz,CH₃NH⁺); 2.85-3.05 (m,2H,CH₂); 3.00-3.40 (m,4H,2CH₂N); 3.66, 3.67, 3.73 and 3.75 (4s,12H,4CH₃O); 3.82-4.00 (m,2H,CH₂CO); 6.65-7.00 (m,6H,Ar); 7.48 (d,2H,J=8.1Hz,Ar); 7.73 (d,2H,J=8.1Hz,Ar); 10.51 (broad band,1H,exch D₂O,NH⁺) ppm.

### Example 13

### N-(3,4-Dimethoxyphenyl)-N-[3[[2-(3,4-dimethoxyphenyl)ethyl]methylamino]propyl]3,5-dinitrobenzamide, hydrochloride

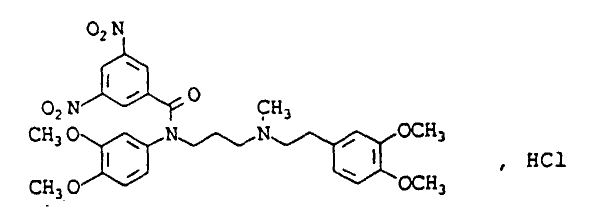

Starting from 1.5 g (2.6 mmol) N-(3,4-dimethoxyphenyl)-N-[3[[2-(3,4-dimethoxyphenyl)ethyl]methylamino]propyl]-3,5-dinitrobenzamide and following the method of salification described in example 4, yielded 1.53 g (95%) of the title compound as yellow solid.
¹H NMR (DMSO-d₆) δ = 1.90-2.10 (m,2H,CH₂); 2.81 (d,3H,J=4.2Hz,CH₃NH⁺); 2.98-3.07 (m,2H,CH₂); 3.02-3.40 (m,4H,2CH₂N); 3.66, 3.67, 3.73 and 3.75 (4s,12H,4CH₃O); 3.88-4.02 (m,2H,CH₂NCO); 6.72-7.00 (m,5H,Ar); 7.16 (s,1H,Ar); 8.57 (s,2H,Ar); 8.70 (s,1H,Ar); 10.50 (broad band,1H,exch D₂O,NH⁺) ppm.

### Example 14

### N-(3,4-Dimethoxyphenyl)-N-[3[[2-(3,4-dimethoxyphenyl)ethyl]cyclopropylamino]propyl]-4-nitrobenzamide, hydrochloride

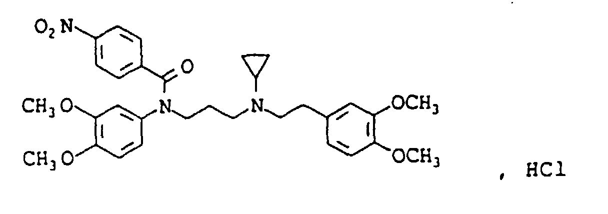

Starting from 0.7 g (1.7 mmol) N-(3,4-dimethoxyphenyl)-N'-[2-(3,4-dimethoxyphenyl)ethyl]-N'-cyclopropyl-1,3-propanediamine (D26) and 0.34 g (1.85 mmol) 4-nitrobenzoyl chloride and following the method described in example 4, yielded 0.555 g (54%) of the title compound as yellow solid. The compound was purified as free base twice by chromatography on silica gel using first 97/3, then 98/2 methylene chloride/methanol, and as hydrochloride by trituration in diethyl ether.
¹H NMR (DMSO-d₆) δ = 0.74-1.00 and 1.00-1.27 (2m,4H,(CH₂)₂); 1.97-2.12 (m,2H,CH₂); 2.78-3.14 (m,3H,CH₂Ar and NCH(CH₂)₂); 3.25-3.44 (m,4H,2CH₂N); 3.65 (s,6H,2CH₃O); 3.73 and 3.75 (2s,6H,2CH₃O); 3.90-4.04 (m,2H,CH₂NCO); 6.68-7.03 (m,6H,Ar); 7.57 (d,2H,J=8.4Hz,Ar); 8.08 (d,2H,J=8.4Hz,Ar); 10.50 (broad band,1H,exch D₂O,NH⁺) ppm.

### Example 15

### N-(4-Hydroxy-3-methoxyphenyl)-N-[3-[[2-(3,4-dimethoxyphenyl)ethyl]methylamino]propyl]-4-nitrobenzamide, hydrochloride

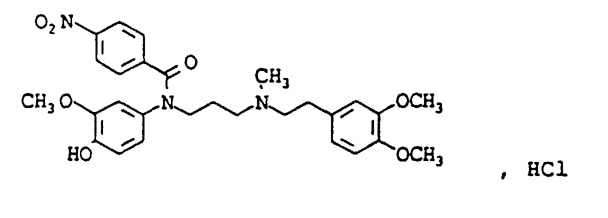

Starting from 500 mg (1.2 mmol) N-(4-hydroxy-3-methoxyphenyl)-N'-[2-(3,4-dimethoxyphenyl)ethyl]-N'-methyl-1,3-propanediamine (D30) and 226 mg (1.2 mmol) 4-nitrobenzoyl chloride and following the method described in example 4, yielded 285 mg (42.4%) of the title compound as yellow crystals. The compound was purified as free base by chromatography on silica using methylene chloride/methanol : 98/2 eluent and as hydrochloride by trituration in diethyl ether. m.p = 109°C
¹H NMR (DMSO-d₆) δ = 1.86-2.10 (m,2H,CH₂); 2.81 (d,3H,J=4.3Hz,CH₃NH+) 2.86-3.00 (m,2H,CH₂); 3.08-3.43 (m,4H,2CH₂N) ; 3.65, 3.73 and 3.75 (3s,9H,3CH₃O); 3.84-3.98 (m,2H,CH₂NCO) ; 6.54-6.95 (m,6H,Ar) ; 7.55 (d,2H,J=8.6Hz,Ar); 8.08 (d,2H,J=8.6Hz,Ar); 9.22 (s,1H,exch D2O,ArOH); 10.03 (broad band,1H,exch D₂O,NH+) ppm

### Example 16

### N-(3,4-Dimethoxyphenyl)-N-[3-[[2-(3,4-dimethoxyphenyl)ethyl]methylamino]propyl]-4-pyridinecarboxamide, dihydrochloride

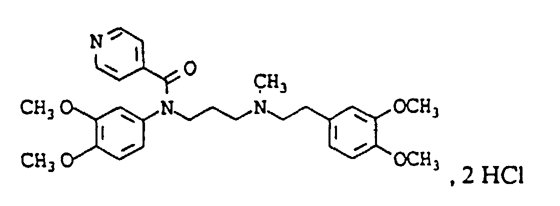

Starting from 1 g of (2.6 mmol) N-(3,4-dimethoxyphenyl)-N'-[2-(3,4-dimethoxyphenyl)ethyl]-N'-methyl-1,3-propanediamine and 0.50 g (2.8 mmol) of 4-pyridine carbonyl chloride, hydrochloride and following the method described in example 4, (except using 2 equivalents of triethylamine), yielded 0.77 g (52.3%) of the title compound as an off yellow solid. The compound was purified as free base by chromatography on silica gel using 95:5 methylene chloride:methanol. m.p = 130-40°C
¹H NMR(DMSO-d₆) δ = 1.86-2.10 (m,2H,CH₂); 2.79 (d,3H,J=3.5Hz,CH₃N); 2.86-3.04 (m,2H,CH₂); 3.07-3.42 (m,4H,2CH₂); 3.67 (s,6H,2CH₃O); 3.72 and 3.75 (2s,6H,2CH₃O); 3.84-4.00 (m,2H,CH₂); 6.70-7.05 (m,6H,Ar); 7.41 (d,2H,J=5.7Hz,Ar); 8.53 (d,2H,J=5.7Hz,Ar); 10.57 (s,1H,exchD₂O,NH⁺) ppm

### Example 17

### N-(3,4-Dimethoxyphenyl)-N-[3-[[2-(3,4-dimethoxyphenyl)ethyl]methylamino]propyl]acetamide, hydrochloride

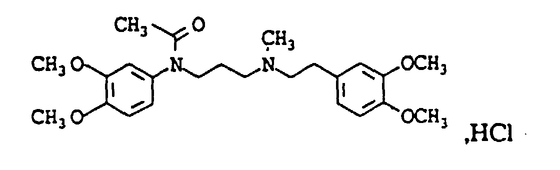

0.13 ml (1.8 mmol) of acetyl chloride were added to an ice cooled stirred solution of 504 mg (1.3 mmol) of N-(3,4-dimethoxyphenyl) N'-[2-(3,4-dimethoxyphenyl)ethyl]-N'-methyl-1,3-propanediamine and 0.198 ml (1.43 mmol) of triethylamine in 20 ml dry methylene chloride. The mixture was stirred for 1 additional hour and poured in water. Methylene chloride was added, the organic phase was washed with a saturated aqueous solution of NaHCO₃, again with water and dried over MgSO₄. The solvent was concentrated in vacuo and the residue was purified by chromatography on silica gel using 93:7 methylene chloride:methanol to give 350 mg of a yellow oil. The compound was salified in methanol by additon of 5.5N anhydrous HCl in diethyl ether and triturated in diethyl ether to afford 290 mg (47.8%) of the title compound as a light beige solid. mp. around 85°C.
¹H NMR(DMSO-d6)δ = 1.75(s,3H,CH₃); 1.76-1.93(m,2H,CH₂); 2;76(d,3H,J=3.7Hz,CH₃); 2.85-3.00(m,2H,CH₂); 3.05-3.48(m,4H,CH₂x2); 3.60-3.80(m,2H,CH₂), 3.72,3.75,3.76,3.77(s,12H,CH₃), 6.70-7.03(m,6H,Ar); 10.49(Broad s, 1H,exch. D₂O,NH) ppm.

### PHARMACOLOGICAL METHODS

### EFFECTS IN THE DOG

**Surgical preparation.** Dogs were anesthetized using 30 mg/kg i.v. sodium pentobarbital. A tracheal cannula was placed for artificial respiration with room air (Harvard 613 pump). A catheter was inserted into the right femoral artery to measure the systemic arterial pressure (Statham P23 ID, GOULD). A 5F Millar microtip catheter pressure transducer was inserted into the left carotid artery and advanced into the left ventricular lumen to measure the left ventricular pressure (LVP) and its first positive derivative dP/dt. Peripheral venous and arterial catheters were placed for drug administration and blood samples collection, respectively. Electrocardiographic variables were measured via a standard lead II ECG. For determination of the Q-T interval of ECG a CV5RL precordial lead of ECG was measured as previously described (Koerner et al.,J.,Cardiovascular Pharmacol. Vol. 16, p. 383-393, 1990). After a left thoracotomy through the fifth intercostal space, the heart was suspended in a pericardial cradle. The left circumflex coronary artery (LCX) was isolated close to its origin and a silk suture was placed around it for later occlusion.

**Experimental time course.** After placement of the silk strand the animal was allowed to stabilize for at least 10 minutes. Arterial blood gases were determined (ABL500, Radiometer), and ventilation was adjusted to maintain these parameters within the normal range. Then, intravenous administration of the drug was performed in 10 minutes. After the drug administration, hemodynamic and ECG variables were again measured and the LCX was occluded for 45 minutes by placing a microclamp. Reperfusion performed by removing the clamp was continued for 5 hours.

**Measurements.** Ventricular arrhythmias were analyzed during the preischemic period, during ischemia and during the first hour of reperfusion. Ventricular premature beats (VPBs) were defined as identifiable premature QRS complexes and salvos as more than 2 consecutive VPBs. Ventricular tachycardia was defined as 4 or more consecutive VPBs. Ventricular fibrillation (VF) was diagnosed when total irregularity of rhythm occurred and when a rate could no longer be measured. If defibrillation was required a DC shock was given.

Arterial blood pressure and ECG were recorded continuously on a Gould polyrecorder. Signals were digitised by an A/D convertor and analysed on an IBM computer using an interactive software (Clodia, Clod sarl) and then transfered to a VAX 4000-200 computer (Digital Equipment Corporation, Maynard, MA) for further analysis. Heart rate was calculated from the ECG. A corrected QT interval was calculated according to the formula of Bazett (Heart 1920; 7: 353-370): QTc = (QT interval in msec) / (R-R interval in sec)^{½}.

### ELECTROPHYSIOLOCAL STUDIES

Guinea pigs (300-350 g) were anesthetized by intravenous injection of sodium pentobarbital (60 mg/kg). After thoracotomy the heart was rapidly excised and placed in oxygenated Tyrode solution. Papillary muscles were removed from the right ventricle. Preparations were then fixed to the silastic base of a 5 ml organ bath and superfused with oxygenated Tyrode solution maintained at 37 ± 1 °C.

The modified Tyrode solution (pH 7.35) contained the following (mM): NaCl 125, KCl 4.0, MgCl₂ 0.5, CaCl₂ 1.8, NaHCO₃ 24, NaH₂PO₄ 0.9 and glucose 5.5. The solution was equilibrated with a gas mixture of 95% O₂ - 5% CO₂.

After a stabilisation period (at least 1 h), transmembrane action potentials were recorded with conventional microelectrodes (10 MOhm) connected to a high input impedance amplifier (BIOLOGIC VF 180). External stimuli were delivered to the preparation with bipolar platinum electrodes placed at one end of the muscle. The pulse duration was 1 ms and the amplitude was twice threshold. The basic cycle length was 1000 ms (PULSAR 6i stimulator). The signals were monitored on a storage oscilloscope (GOULD 1602) and simultaneously recorded on a digital tape recorder (BIOLOGIC DTR 1200) for further analysis.

Measurements were made of resting membrane potential (RMP), action potential amplitude (APA) and action potential durations at 30, 50 and 90% repolarization (APD₃₀, APD₅₀ and APD₉₀ respectively). Recordings were made after 30 min of equilibration for each concentration. Only recordings in which the same impalement was maintained throughout the entire experiment were used for analysis.

### STATISTICAL ANALYSIS

Values were expressed as mean ± SEM and were compared using analysis of variance followed by Duncan's new multiple range test or Student's t-test as appropriate when the variables where found to be Gaussian distributed. For the duration of VT and VF and the number of VPBs which were not found Gaussian-distributed, medians were compared using the Mann-Withney test. Binomially distributed variables such as incidence of VT and VF and mortality were compared using X² for a 2 x n table, followed by Fisher exact tests to compare individual groups. All statistical comparisons were performed using RS/1 release 4 software (BBN Software Product Corporation, Cambridge, MA) on the VAX 4000-200 computer. A p value less than 0.05 was considered statistically significant.

### RESULTS

### EFFECTS IN ANESTHETIZED DOGS.

### Hemodynamic and electrocardiographic parameters.

The effects of Compound I (0.3 and 1.0 mg/kg) in anesthetized dogs were compared to those of d-sotalol (3.0 and 10.0 mg/kg). At the end of the stabilisation period, before drug administration, there was no difference between the groups in the hemodynamic parameters. During the preischemic period, both compound reduced heart rate in a dose-dependent manner. With only minimal changes in mean arterial and left ventricular pressure, Compound I and d-sotalol both reduced the pressure rate product. This effect was associated for both compounds with a reduction in the left ventricular dP/dtmax. Similar changes in hemodynamic parameters were also observed both during the ischemic period and after 60 min of reperfusion.

During the preischemic period both Compound I and d-Sotalol prolonged QT and QTc intervals (table 1) but did not change the PR interval. Although a similar prolongation of QT interval was also observed during ischemia, a lesser effect was noted during the reperfusion period. During ischemia the high doses of Compound I and d-sotalol both increased QTc interval and this action was abolished during the reperfusion. Except for the lower dose of Compound I during the ischemic period, there was no marked change in the PR interval duration.

### Ischemia and reperfusion-induced arrhythmias.

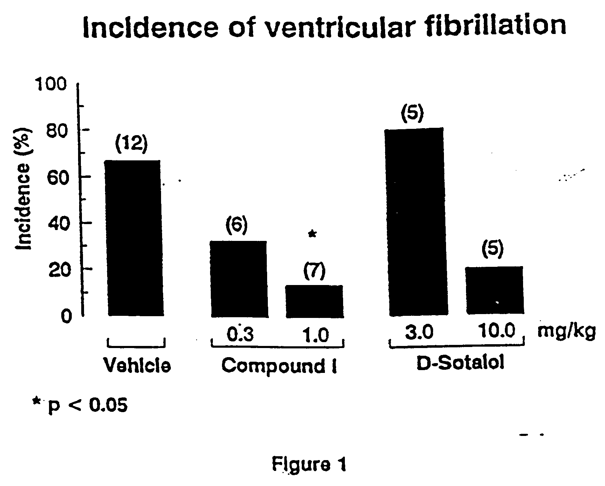

The effects of Compound I (0.3 and 1 mgKg) were compared to those of D-sotalol (3 and 10 mg/kg). Ventricular arrhythmias were analyzed during ischemia, maintained for 45 min, and again during the first 60 min of the reperfusion period. The effects of Compound I and D-sotalol on the incidence of ventricular fibrillation observed during these period were summarized in figure 1. The results show that 66.7 % of the control dogs (8 out of 12 dogs) exhibited ventricular fibrillation during either ischemia (2 dogs) or reperfusion (4 dogs). The incidence of ventricular fibrillation was reduced to 33.3 % and 14.3 % (p <0.05) in presence of Compound I (0.3 or 1.0 mg/kg, respectively), and none of the dogs treated with Compound I experienced ventricular fibrillation during ischemia. In comparison, only the high dose of D-sotalol (10.0 mg/kg) reduced the incidence of ventricular fibrillation (figure 2), and one dog treated with 3.0 mg/kg showed ventricular fibrillation during the ischemic period.

### EFFECTS OF COMPOUND I ON ACTION POTENTIAL CHARACTERISTICS OF GUINEA PIG MUSCLE.

The most prominent effect of sotalol (3 to 100 µM) was a dose-dependent increase in action potential duration. The results summarized in table 2 show that Compound I induced a biphasic effect on action potential duration, depending on the concentration. An action potential prolongation was observed with Compound I at low concentrations (0.3 and 1 µM). However, when the concentration was further increased (3 µM) there was no further increase in APD. At a higher concentration (10 µM) Compound I reduced action potential duration.

The results summarized in table 2 show that both Compound I and d-Sotalol did not change the resting membrane potential, the action potential amplitude, and the Vmax, even at the highest concentrations. Table 3 summarises the results obtained from these experiments when repeated using the test compounds indicated therein.

**Table 1**

| Effect of Compound I and d-Sotalol on electrocardiographic parameters measured in anesthetized dog. | | | | | | |
|---|---|---|---|---|---|---|
| | | n | Before drug | After drug | Ischemia (45 min) | reperfusion (60 min) |
| PR (msec) | | | | | | |
| Vehicle | | 7 | 116 ± 4 | 116 ± 3 | 116 ± 4 | 110 ± 1 |
| Compound I | 0.3 mg/kg | 6 | 111 ± 4 | 112 ± 4 | 120 ± 6 ** | 115 ± 7 |
| | 1.0 mg/kg | 7 | 115 ± 3 | 116 ± 3 | 113 ± 3 | 116 ± 3 |
| d-Sotalol | 3.0 mg/kg | 5 | 105 ± 5 | 118 ± 7 | 111 ± 7 | 112 ± 5 |
| | 10.0 mg/kg | 5 | 109 ± 5 | 115 ± 5 | 113 ± 5 | 113 ± 6 |

| QT (msec) | | | | | | |
|---|---|---|---|---|---|---|
| Vehicle | | 7 | 252 ± 12 | 250 ± 10 | 250 ± 6 | 245 ± 12 |
| Compound I | 0.3 mg/kg | 6 | 245 ± 16 | 280 ± 18 *** | 268 ± 18 *** | 262 ± 19 |
| | 1.0 mg/kg | 7 | 243 ± 11 | 295 ± 17 §*** | 293 ± 19 §*** | 267 ± 11 *** |
| d-Sotalol | 3.0 mg/kg | 5 | 238 ± 11 | 269 ± 12 *** | 264 ± 7 ** | 251 ± 11 |
| | 10.0 mg/kg | 5 | 251 ± 11 | 337 ± 19 §** | 312 ± 14 §** | 285 ± 11 * |

| QTc (msec/sec^{½}) | | | | | | |
|---|---|---|---|---|---|---|
| Vehicle | | 7 | 397 ± 11 | 393 ± 11 | 389 ± 11 | 394 ± 11 |
| Compound I | 0.3 mg/kg | 6 | 388 ± 12 | 422 ± 16 ** | 398 ± 11 | 393 ± 13 |
| | 1.0 mg/kg | 7 | 388 ± 8 | 432 ± 17 ** | 420 ± 16 ** | 400 ± 11 |
| d-Sotalol | 3.0 mg/kg | 5 | 384 ± 8 | 397 ± 11 * | 400 ± 8 | 392 ± 16 |
| | 10.0 mg/kg | 5 | 386 ± 3 | 440 ± 14 §* | 423 ± 10 * | 400 ± 8 |
| Values represent mean ± SEM of n experiments. Analysis of variance followed by Duncan's new multilple range test or Student's t test for paired values were used as appropriate. | | | | | | |

| | | | | | | |
|---|---|---|---|---|---|---|
| * < p 0.05, | | | | | | |
| ** p < 0.01, | | | | | | |
| *** p < 0.001 vs before drug values, | | | | | | |
| § p < 0.05 vs vehicle group. | | | | | | |

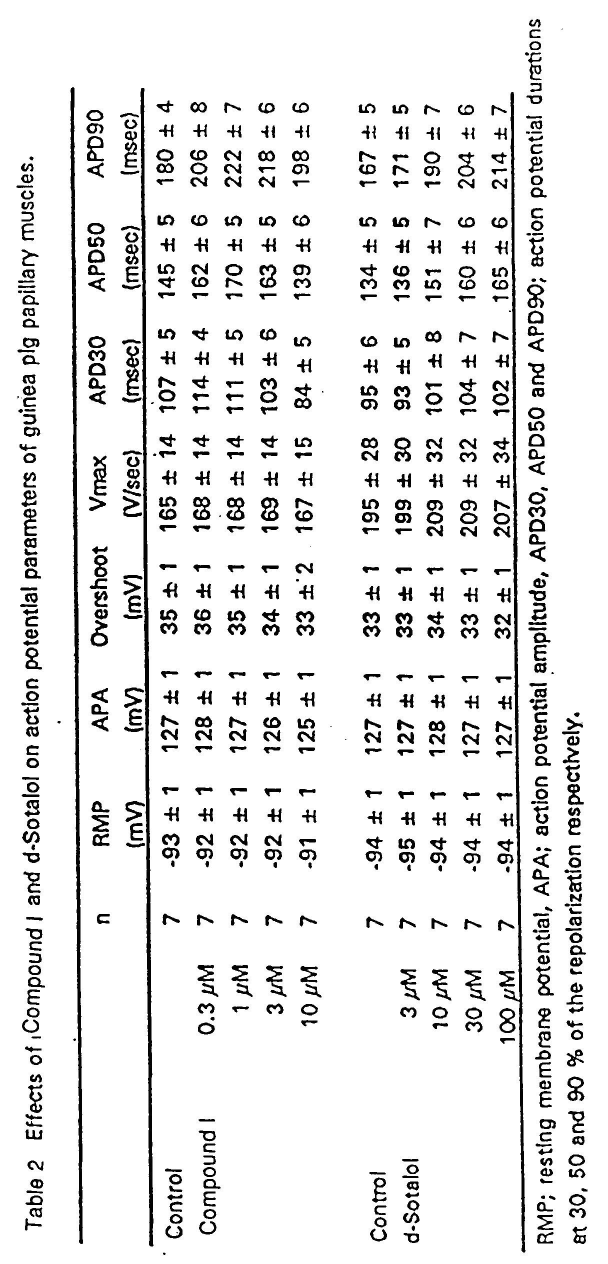

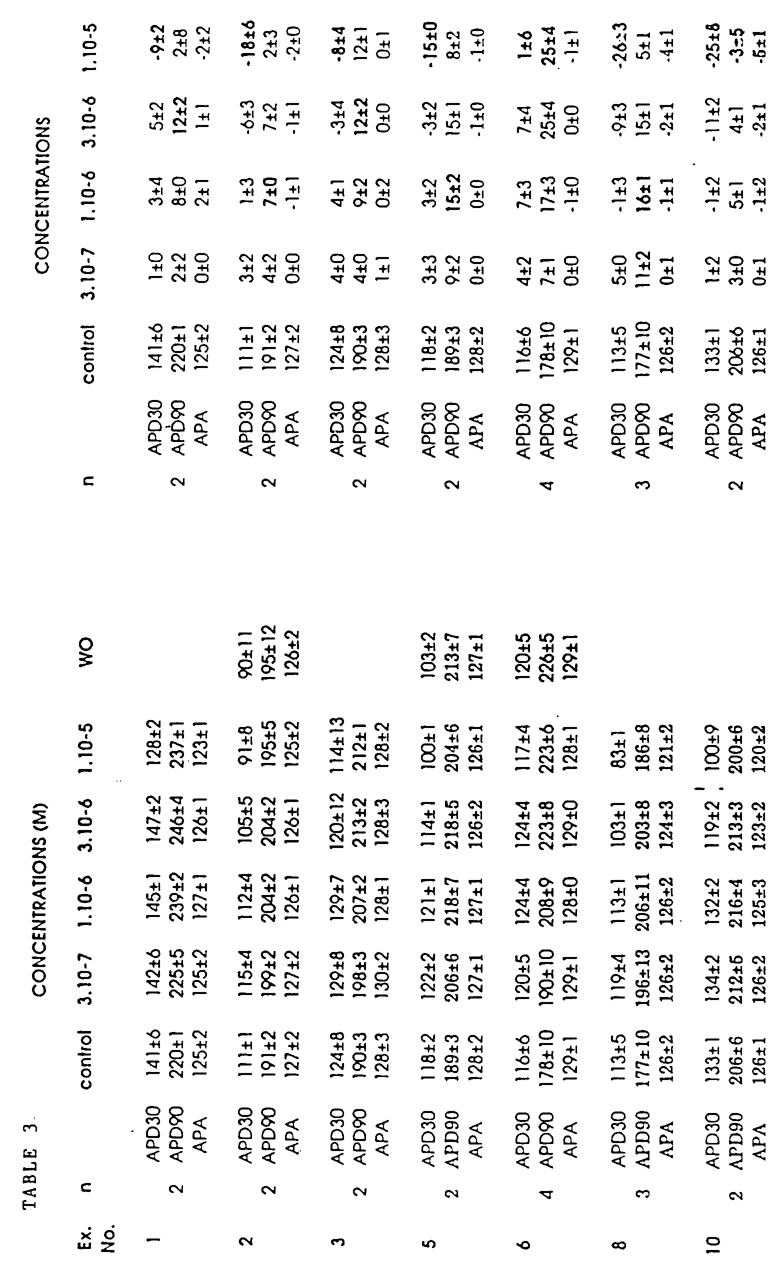

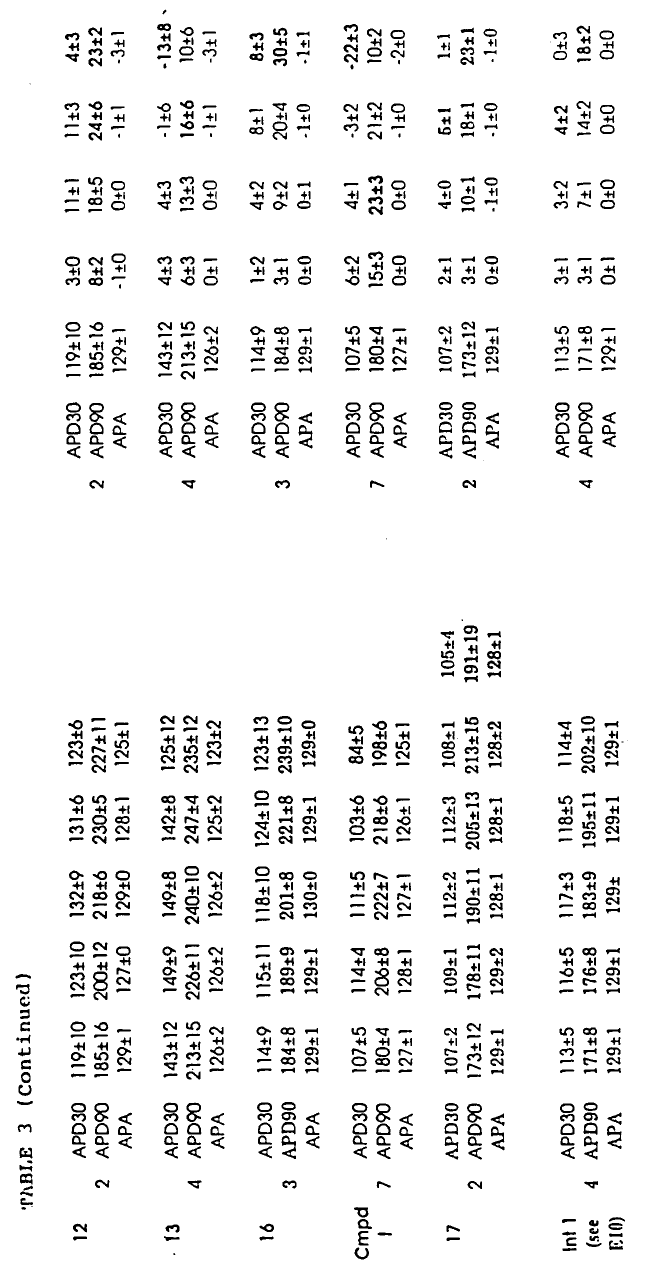

## Claims

1. A pharmaceutical composition for the treatment of cardiac arrhythmia and ischaemic rhythm disorders, which composition comprises an effective, non-toxic amount of a compound having combined Class III and Class IV anti-arrhythmic activity and a pharmaceutically acceptable carrier therefor.

2. A composition according to claim 1, for the treatment of ventricular fibrillation..

3. A composition according to claim 1, for the treatment of atrial fibrillation and flutter.

4. A compound having combined Class III and Class IV anti-arrhythmic activity for use in the treatment of cardiac arrhythmia and ischaemic rhythm disorders.

5. A compound according to claim 4, for the treatment of ventricular fibrillation.

6. A compound according to claim 4, for the treatment of atrial fibrillation and flutter.

7. The use of a compound having combined Class III and Class IV anti-arrhythmic activity for the manufacture of a medicament for the treatment of cardiac arrhythmia and ischaemic rhythm disorders.

8. A use according to claim 7, wherein the medicament is for the treatment of ventricular fibrillation..

9. A use according to claim 7, for the treatment of atrial fibrillation and flutter.
